(19) Europäisches Patentamt
European Patent Office
Office européen des brevets

(11) Veröffentlichungsnummer: **0 021 324**
**B1**

# (12) EUROPÄISCHE PATENTSCHRIFT

(45) Veröffentlichungstag der Patentschrift: **20.01.82**

(51) Int. Cl.[3]: **C 07 C 125/063,** C 07 C 155/00, C 07 D 213/64, A 01 N 47/20

(21) Anmeldenummer: **80103371.3**

(22) Anmeldetag: **18.06.80**

(54) **m-Anilidurethane und diese enthaltende Herbizide.**

(30) Priorität: **28.06.79 DE 2926049**

(43) Veröffentlichungstag der Anmeldung: **07.01.81 Patentblatt 81/1**

(45) Bekanntmachung des Hinweises auf die Patenterteilung: **20.01.82 Patentblatt 82/3**

(84) Benannte Vertragsstaaten: **AT BE CH DE FR GB IT LI NL SE**

(56) Entgegenhaltungen: **GB-A-1 315 871**

(73) Patentinhaber: **BASF Aktiengesellschaft, Carl-Bosch-Strasse 38, D-6700 Ludwigshafen (DE)**

(72) Erfinder: **Schirmer, Ulrich, Dr., Berghalde 79, D-6900 Heidelberg 1 (DE)**
Erfinder: **Becker, Rainer, Dr., Sonnenwendstrasse 83, D-6702 Bad Duerkheim 1 (DE)**
Erfinder: **Wuerzer, Bruno, Dr., Wilhelm-Busch-Strasse 55, D-6703 Limburgerhof (DE)**

**EP 0 021 324 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# m-Anilidurethane und diese enthaltende Herbizide

Die vorliegende Erfindung betrifft neue m-Anilidurethane, Verfahren zur Herstellung dieser m-Anilidurethane, Herbizide, welche diese Verbindungen enthalten, sowie Verfahren zur Bekämpfung unerwünschten Pflanzenwuchses mit diesen Verbindungen.

Es ist bekannt, m-Anilidoharnstoffe, z.B. das 2,4-Dichlorphenoxyessigsäure-3′-(N′-dimethylureido)-anilid als Herbizid zur Bekämfpung unerwünschter breitblättriger und grasartiger Unkräuter zu verwenden. Ein Hinweis auf die Verwendung als selektive herbizide Mittel ist aber aus der Literatur nicht zu entnehmen (DE-AS 1793226). Ausserdem werden in der US-PS 3979 202 zahlreiche 3′-(Carbamoyloxy)-anilide, z.B. das 3′-N-Isopropyl-carbamoyl-oxy-propionsäureanilid, mit recht divergierender herbizider Wirksamkeit gegenüber höheren Pflanzen offenbart. Es ist ferner bekannt, dass Phenoxyphenoxycarbonsäureamide (DE-OS 2632581) eine herbizide Wirkung besonders gegen dikotyle Unkräuter entfalten. Ausserdem sind aus der DE-OS 2531643 Phenoxyphenoxycarbonsäureamide bekannt, die eine herbizide Wirkung gegen monokotyle Pflanzen zeigen, während dikotyle Kulturpflanzen nicht geschädigt werden. Aus den DE-OS 2725146 und 2703838 sind m-Anilidurethane mit selektiver herbizider Wirkung bekannt.

Es wurde nun gefunden, dass m-Anilidurethane der Formel

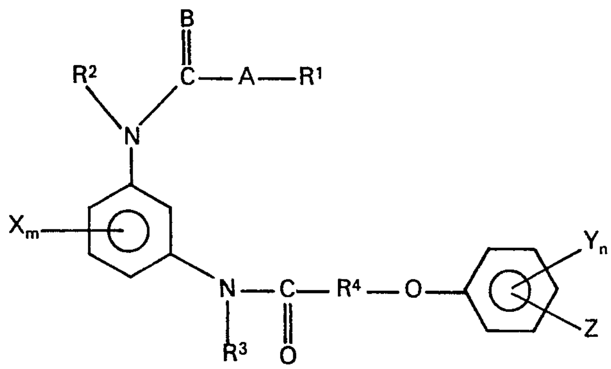

in der

A und B unabhängig voneinander Sauerstoff oder Schwefel

$R^1$ ggf. durch Halogen oder Alkoxy oder Alkoxycarbonyl oder Cyan substituiertes Alkyl oder ggf. durch Halogen substituiertes Alkenyl oder ggf. durch Halogen oder Alkoxy substituiertes Alkinyl oder ggf. durch Alkyl substituiertes Cycloalkyl oder Aryl

$R^2$ und $R^3$ jeweils unabhängig voneinander Wasserstoff, Alkyl, Alkoxyalkyl, Halogenalkyl

$R^4$ einen ggf. durch Alkyl, Alkoxyalkyl oder Halogenalkyl substituierten Alkylrest mit 1 bis 6 Kohlenstoffatomen

X Wasserstoff, Alkyl, Halogenalkyl, Alkoxy, Halogen, Nitro oder Amino

Y Wasserstoff, Alkyl, Halogenalkyl, Alkoxyalkyl, Cycloalkyl, Aralkyl, Halogen, Alkoxy, Halogenalkyl, Alkylthio, Nitro, Aryl, Thiocyanato,

Cyano, 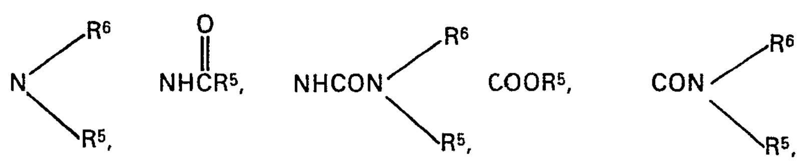 $COOR^5$,

$SO_2R^5$, $OSO_2R^5$, $COR^5$, 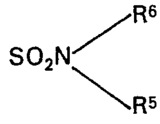 bedeutet,

wobei $R^5$ und $R^6$ jeweils unabhängig voneinander Wasserstoff bedeuten oder die für $R^1$ genannten Bedeutung haben,

Z den Rest 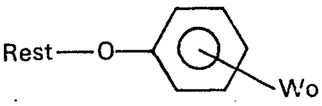 oder 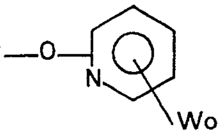 bedeutet,

wobei W unabhängig von Y die für Y genannten Bedeutungen hat und m, n und o die Zahlen 1 bis 4 bedeuten, eine gute herbizide Wirkung gegen unerwünschte Pflanzen haben. Hervorzuheben ist hierbei die Wirksamkeit gegen unerwünschte Pflanzen aus der Familie der Gramineen (Ungräser). Gleichzeitig sind wichtige Kulturpflanzen in hohem Masse unempfindlich gegenüber den neuen Wirkstoffen.

Die in der allgemeinen Formel angeführten Reste können beispielsweise folgende Bedeutungen haben:

$R^1$ unsubstituiertes Alkyl oder durch Halogen oder Alkoxy oder Alkoxycarbonyl oder Cyan substituiertes Alkyl (z.B. Methyl, Äthyl, 2-Chloräthyl, 2-Methoxyäthyl, Methoxycarbonylmethyl, Isopropyl, n-Propyl, n-Butyl, sec-Butyl, tert.-Butyl, Cyanomethyl) oder ggf. durch Halogen substituiertes Alkenyl (z.B. Allyl, 1-Chlorpropen(1)yl(3), Buten(1)yl(3)), oder ggf. durch Halogen oder Alkoxy substituiertes Alkinyl (z.B. Propargyl, Butin(1)yl(3), 1-Chlorbutin(2)yl(4)) oder ggf. durch Alkyl substituiertes Cycloalkyl (z.B. Cyclopentyl, Cyclohexyl, 3-Methylcyclohexyl, 2,6-Dimethylcyclohexyl, Cycloheptyl, 4-tert.-Butyl-cyclohexyl) oder Aryl (z.B. Phenyl),

$R^2$ und $R^3$ jeweils unabhängig voneinander Wasserstoff, Alkyl (z.B. Methyl, Äthyl, iso-Propyl), Alkoxyalkyl (z.B. Methoxymethyl, 2-Methoxyäthyl), Halogenalkyl (z.B. Chlormethyl, 2-Chloräthyl),

$R^4$ einen ggf. durch Alkyl, Alkoxyalkyl oder Halogenalkyl substituierten Alkylrest mit 1 bis 6 Kohlenstoffatomen (z.B. Methylen, Methylmethylen, Dimethylmethylen, Propylen, Hexylen, Chlormethylmethylen, Methoxmethylmethylen, Äthylmethylen, Methyläthylen, Isopropylmethylen, 1-Chlormethyläthylen, Methoxymethylmethylen, 2-Chloräthylmethylen, Diäthylmethylen, Äthylen, Methylpropylen, Butylen, Dimethyläthylen, Propylmethylen)

X Wasserstoff, Alkyl (z.B. Methyl), Halogenalkyl (z.B. Trifluormethyl), Alkoxy (z.B. Methoxy), Halogen (z.B. Fluor, Chlor, Brom oder Jod), Nitro oder Amino,

Y und W jeweils unabhängig voneinander Wasserstoff, Alkyl (z.B. Methyl, Isopropyl), Halogenalkyl (z.B. Trifluormethyl), Alkoxyalkyl (z.B. Methoxymethyl), Cycloalkyl (z.B. Cyclohexyl), Aralkyl (z.B. Benzyl), Halogen (z.B. Fluor, Chlor, Brom oder Jod), Alkoxy (z.B. Methoxy), Halogenalkyl (z.B. Trifluormethoxy), Alkylthio (z.B. Methylthio), Nitro, Aryl (z.B. Phenyl), Thiocyanato, Cyano,

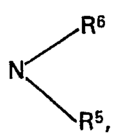 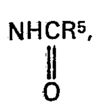 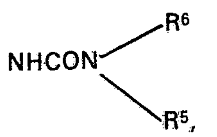 $COOR^5$, 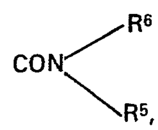 $SO_2R^5$, $COR^5$,

$OSO_2R^5$, 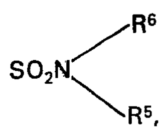 wobei $R^5$ und $R^6$ jeweils unabhängig voneinander Wasserstoff bedeutet oder die für $R^1$ genannte Bedeutung hat.

Die neuen Verbindungen können beispielsweise nach folgenden Verfahren hergestellt werden, wobei die Reste A, B, $R^1$, $R^2$, $R^3$, $R^4$, X, Y, Z, m und n die oben angegebene Bedeutung haben. Wenn im folgenden von Urethanen und Chlorameisensäureestern die Rede ist, sollen unter diesen beiden Sammelbegriffen auch Thiono-, Thio- und Dithiourethane sowie Chlorameisensäurethionoester, Chlorameisensäurethioester und Chlorameisensäuredithioester verstanden werden.

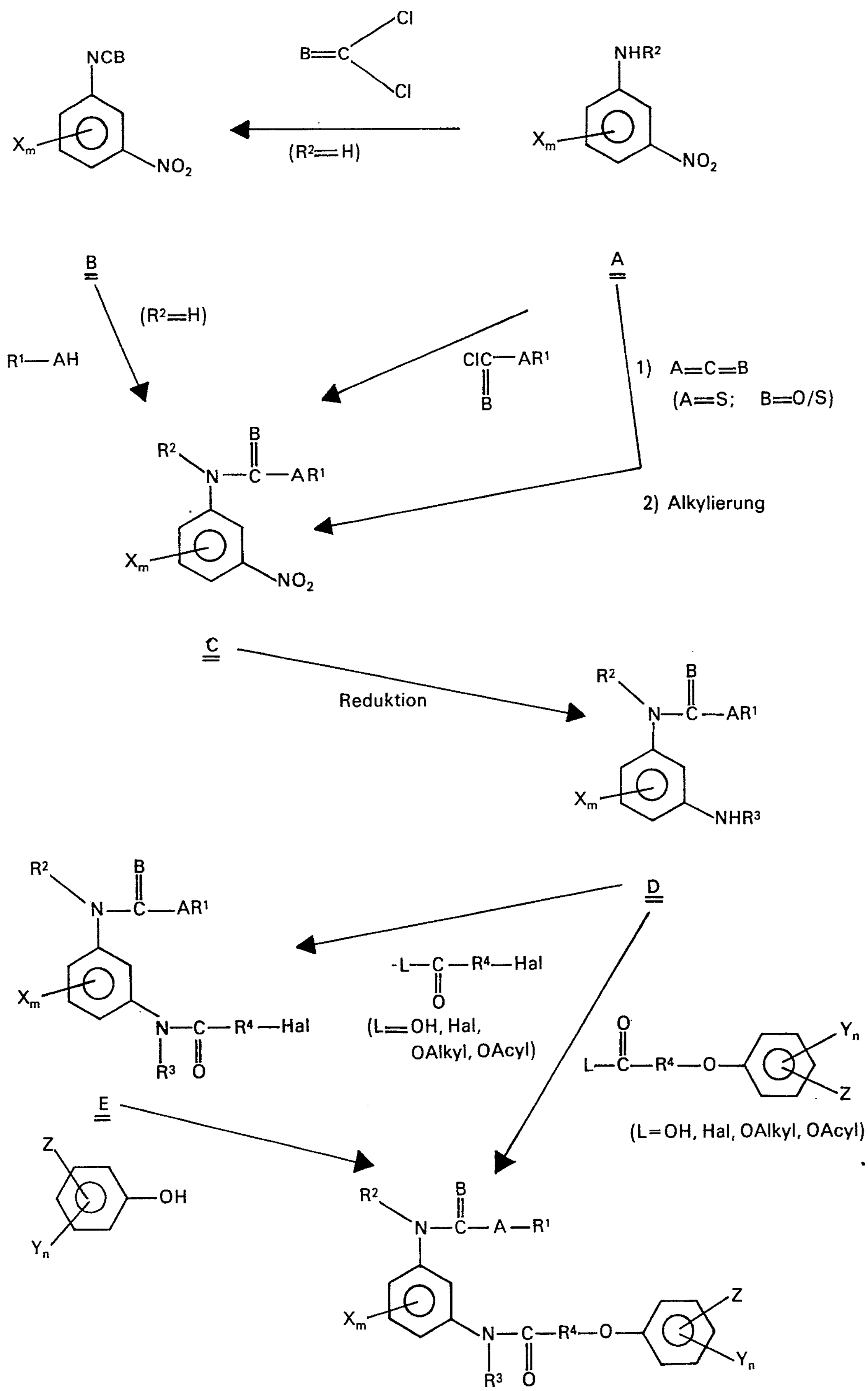
(R$^2$=H)
A
B
(R$^2$=H)
R$^1$—AH
1) A=C=B
(A=S; B=O/S)
2) Alkylierung
C
Reduktion
D
(L=OH, Hal, OAlkyl, OAcyl)
(L=OH, Hal, OAlkyl, OAcyl)
E

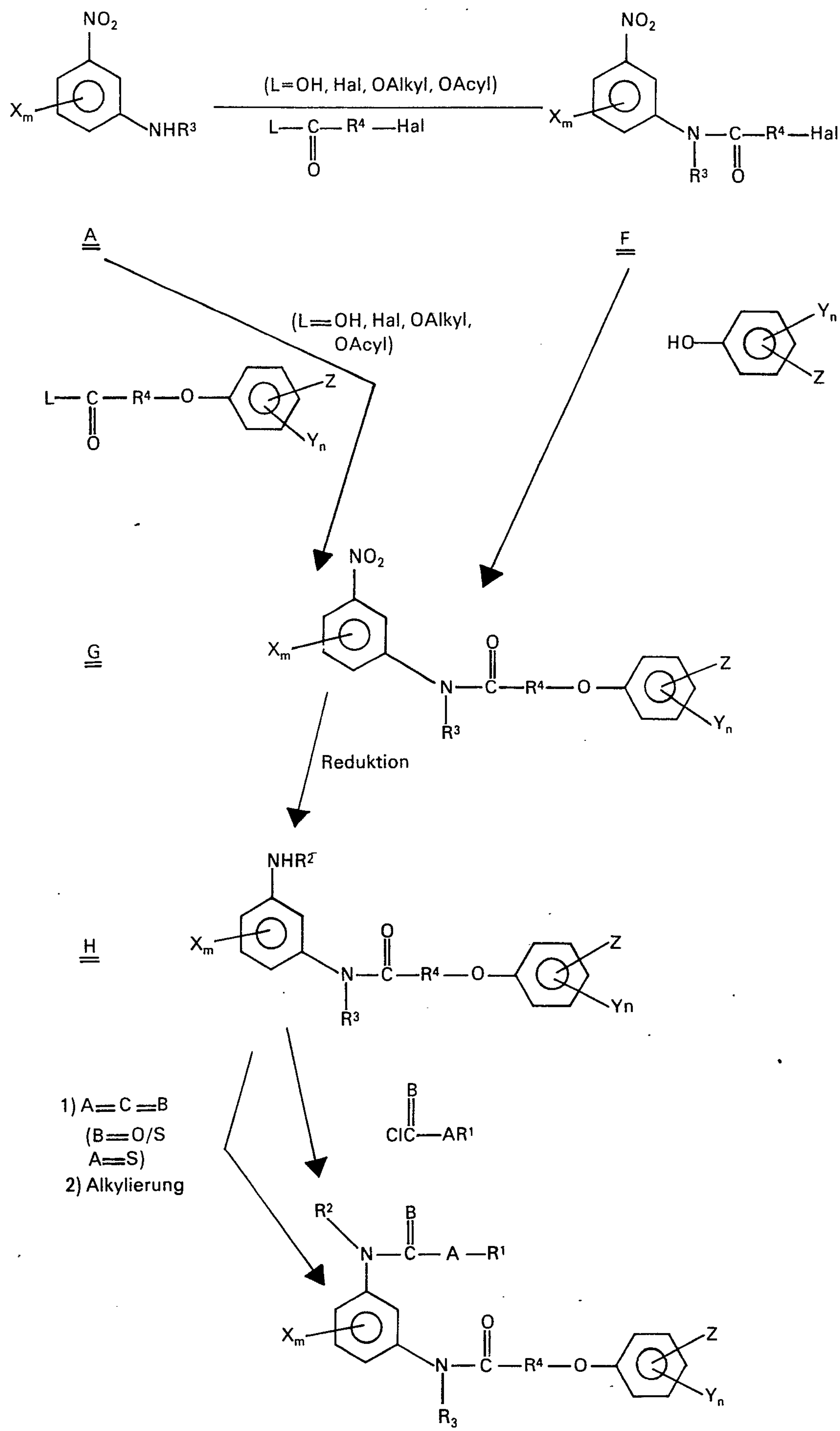
(L=OH, Hal, OAlkyl, OAcyl)
A
F
(L=OH, Hal, OAlkyl, OAcyl)
G
Reduktion
H
1) A=C=B
(B=O/S
A=S)
2) Alkylierung

Aus den aufgezeigten Reaktionsschemata gehen eindeutig die wechselseitigen Beziehungen zwischen den Ausgangsstoffen hervor. Weiterhin wird deutlich, dass je nach Natur der Substituenten A, B, $R^1$, $R^2$, $R^3$, $R^4$, X, Y und Z sowie der Verfügbarkeit der jeweiligen Reaktionspartner der eine oder andere Weg vorteilhaft sein kann.

Ausgehend von bekannten meta-Nitranilinen (A) lassen sich meta-Nitrophenyl-iso(thio)cyanate (B) herstellen (W. Siefken, J. Liebigs Annalen der Chemie 562, 75 ff, (1949)), die ihrerseits mit den Komponenten $R^1$-AH glatt zu den Nitro(thio) urethanen (C) reagieren (S. Petersen in Methoden der Organ. Chemie, Band VIII, Seite 131, Georg-Thieme-Verlag, Stuttgart, 4. Auflage, (1952)), die allerdings auch direkt aus den meta-Nitranilinen (A) mit Chlorameisensäureestern ($R^1$A-CB-Cl) (DZ-OS 1643763) oder mit Schwefelkohlenstoff bzw. Kohlenoxysulfid, Base und Alkylierungsmittel (Methoden der Organ. Chemie, Band IX, Seite 831 f, Georg-Thieme-Verlag, Stuttgart, 4. Auflage, 1955)) zugänglich sind. Nachfolgende Reduktion führt zu den Aminourethanen (D, $R^3$ = H) (S. Schröter in Methoden der Organ. Chemie, Band XI/1, Seite 350 ff, Georg-Thieme-Verlag, Stuttgart, 4. Auflage 1957) die entweder direkt oder nach erfolgter Umwandlung in das Aminostickstoff monosubstituierte Produkt (D, $R^3 \neq$ H) (Methoden der Organ. Chemie, Band XI/1, Seite 24 ff, Georg-Thieme-Verlag, Stuttgart, 4. Auflage 1957) mit Phenoxycarbonsäuren, -säurehalogeniden, -säureestern oder -säureanhydriden zu den erfindungsgemässen m-Anilidurethanen acyliert werden (Methoden der Organ. Chemie, Band XI/2, Seite 3 ff, Georg-Thieme-Verlag, Stuttgart, 4. Auflage, 1958).

Die Aminourethane (D) lassen sich auch zuerst mit einer Halogencarbonsäure, -säurehalogenid, -säureester oder -säureanhydrid zu den m-Anilidurethanen (E) umsetzen, die dann mit Phenolen zu den erfindungsgemässen m-Anilidurethanen reagieren (Methoden der Organ. Chemie, Band VI/3, Seite 54 f, Georg-Thieme-Verlag, Stuttgart, 4. Auflage 1965).

Eine weitere Synthesemöglichkeit besteht in der Reaktion der m-Nitroaniline (A) mit Phenoxycarbonsäuren, -säurehalogeniden, -säureestern oder -säureanhydriden zu den m-Nitroaniliden (G), die auch über die Nitrohalogenide (F) zugänglich sind.

Die Reduktion der m-Nitroanilide (G) führt zu den m-Aminoaniliden (H, $R^2$ = H), die entweder direkt oder nach erfolgter Umwandlung in das am Aminostickstoff monosubstituierte Produkt (H, $R^2 \neq$ H) mit Chlorameisensäureestern ($R^1$ACB-Cl) oder mit Schwefelkohlenstoff bzw. Kohlenoxysulfid, Base und Alkylierungsmittel zu den erfindungsgemässen m-Anilidurethanen umgesetzt werden.

Nachfolgend werden die bevorzugten Syntheseschritte genauer beschrieben:

a) Die Umsetzung von 3-Nitrophenyliso(thio)-cyanaten (B) erfolgt mit oder ohne einem für Iso(thio)cyanatreaktionen gebräuchlichen Katalysator, z.B. tert. Amine (Triäthylamin, 1,4-Diazabicyclo-(2,2,2)-octan), stickstoffhaltige Heterocyclen (Pyridin, 1,2-Dimethylimidazol) oder organische Zinnverbindungen (Dibutylzinndiacetat, Dimethylzinndichlorid) gegebenenfalls in einem unter den Reaktionsbedingungen inerten Lösungsmittel, z.B. Kohlenwasserstoffe (Ligroin, Benzin, Tuluol, Pentan, Cyclohexan), Halogenkohlenwasserstoffe (Methylenchlorid, Chloroform, Dichloräthan, Chlorbenzol, o-, m- oder p-Dichlorbenzol), Nitrokohlenwasserstoffe (Nitrobenzol, Nitromethan), Nitrile (Acetonitril, Butyronitril, Benzonitril), Äther (Diäthyläther, Tetrahydrofuran, Dioxan), Ester (Essigsäureäthylester, Propionsäuremethylester, Ketone (Aceton, Methyläthylketon) oder Amide (Dimethylformamid, Foramid) DE-OS 1568138) bei Temperaturen im Bereich von 0 bis 150 °C vorzugsweise im Bereich von 40 bis 100 °C.

b) 3-Nitraniline (A) bzw. 3-Aminoanilide (H) werden mit Chlorameisensäureester in einem geeigneten Lösungsmittel, z.B. Wasser, Alkohole (Methanol, Äthanol, Isopropanol) oder wie unter a) angegeben, unter Zuhilfenahme eines üblichen Säurebinders, z.B. Alkalihydroxide, -carbonate, -hydrogencarbonate, Erdalkalioxide, -hydroxide, -carbonate, -hydrogencarbonate, tertiäre organische Basen (z.B. Triäthylamin, Pyridin, N, n-Dimethylamin, N, n-Dimethylcyclohexylamin, Chinolin, Tributylamin) oder Ausgangsprodukt 3-Nitranilin, bei Temperaturen von −20 °C bis 150 °C, vorzugsweise im Bereich von 20 bis 80 °C umgesetzt.

c) Die Reduktion der Nitrourethane (C) bzw. Nitroanilide kann nach einem der bekannten Verfahren durchgeführt werden, beispielsweise durch katalytische Hydrierung, durch eine Metall-Säure-Kombination, z.B. eine Kombination Eisen-Säure, durch eine Metall-Alkohol-Kombination, z.B. Zinkstaub-wässriger Alkohol, Eisenwässriger Alkohol.

d) 3-Nitroaniline (A) bzw. Aminourethane (D) werden mit Phenoxycarbonsäurehalogeniden bzw. mit Halogencarbonsäurehalogeniden in einem geeigneten Lösungsmittel, unter Zuhilfenahme eines üblichen Säurebinders, wie unter a) angegeben, bei Temperaturen von −20 bis 150 °C, vorzugsweise bei -60 °C umgesetzt. Statt den Säurehalogeniden lassen sich auch die Säuren selbst einsetzen, wenn man diese mit einem aliphatischen Carbodiimid, z.B. Dicyclohexylcarbondiimid, aktiviert. Von den unter a) angegebenen Lösungsmitteln eignen sich Äther, z.B Tetrahydrofuran, besonders gut, wobei vorzugsweise im Bereich von 0–60 °C gearbeitet wird.

Die Reaktion der 3-Nitraniline (A) bzw. Aminourethane (D) mit Phenoxycarbonsäureestern führt man entweder ohne Lösungsmittel oder mit einem indifferenten Lösungsmittel wie Kohlenwasserstoffe (Toluol), Halogenkohlenwasserstoffe (Dichlorbenzol) oder Amide (Dimethylformamid) bei Temperaturen von 50–180 °C, vorzugsweise bei 80–150 °C durch.

e) Die Halogenamidurethane (E) bzw. die Nitrohalogenamide (F) werden entweder mit Alkaliphenolaten in einem indifferenten Lösungsmittel wie unter a) angegeben oder mit feingepulvertem Kaliumcarbonat und Phenol in einem Keton (Aceton, Mehyläthylketon) bei Temperaturen im Bereich von 0 bis 150 °C, vorzugsweise im Bereich von 40–100 °C umgesetzt.

Die folgenden Beispiele sollen die Herstellung der neuen m-Anilidurethane und ihrer Vorprodukte erläutern:

I. Nitrourethane

Beispiel A

Zu 138 Gew.-Teilen m-Nitranilin in 500 Gew.-Teilen Tetrahydrofuran (THF) werden 87 Gew.-Teile Natriumhydrogencarbonat gegeben. Unter Rühren tropft man bei Raumtemperatur 120 Gew.-Teile Chlorameisensäurethiomethylester zu, lässt 16 Stunden bei Raumtemperatur nachrühren, filtriert, destilliert das Lösungsmittel am Rotationsverdampfer ab und rührt das erhaltene Öl in Toluol ein. Die sich abscheidenden Kristalle werden abgesaugt und getrocknet:

Fp.: 137–138 °C.

Die Verbindung hat folgende Strukturformel:

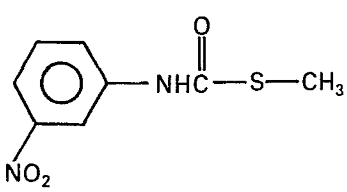

Beispiel B

Zu 112 Gew.-Teilen 3-Nitrophenylisocyanat in 600 Gew.-Teilen Toluol werden 51 Gew.-Teile tert. Butanol gegeben. Nach 4 Stunden gibt man einige Tropfen Triäthylamin zu und lässt 48 Stunden stehen. Nach dem Abziehen des Lösungsmittels im Vakuum erhält man weisse Kristalle.

Fp.: 97–99 °C.

Die Verbindung hat folgende Strukturformel:

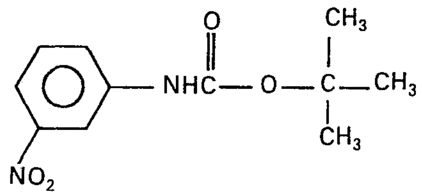

Nach entsprechenden Verfahren können folgende Nitrourethane (C) hergestellt werden:

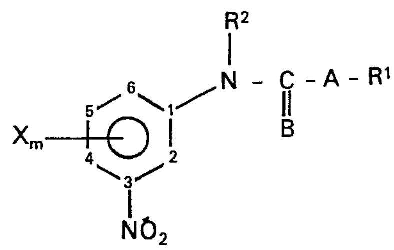

| A | B | X | $R^1$ | $R^2$ | Fp °C |
|---|---|---|---|---|---|
| O | O | H | $CH_3$ | H | 153–155 |
| O | S | H | $CH_3$ | H | |
| O | O | 6-$CH_3$ | $CH_3$ | H | 132–133 |
| O | O | H | Phenyl | H | 123–125 |
| O | O | $H_3$ | Phenyl | $CH_3$ | 69–70 |
| O | O | 6-F | Phenyl | H | 138–140 |
| O | O | 5-$CF_3$ | $CH_3$ | H | 86–87 |
| O | O | 6-$CH_3$ | $C_2H_5$ | H | 131–133 |
| O | O | H | $C_2H_5$ | H | 64–66 |
| O | O | 2-$CH_3$ | Phenyl | H | 112–114 |
| O | O | 4-$CH_3$ | $CH_3$ | H | 114–117 |
| O | O | H | Cyclooctyl | H | 103–105 |
| O | O | H | $CH_2COOCH_3$ | H | 123–125 |
| O | O | 5-$CF_3$ | $CH(CH_3)_2$ | H | 121–123 |
| O | O | H | $CH_3$ | $CH_3$ | 58–61 |
| O | O | H | 3,5-Dimethylcyclohexyl | H | 128–129 |
| O | O | H | $CH(CH_3)_2$ | H | 86–88 |
| O | O | 6F | $CH_3$ | H | 116–118 |
| O | O | 4Cl | Phenyl | H | 125–127 |
| O | O | 4Cl | $CH_3$ | H | 122–124 |
| O | O | 4-$CH_3$ | $C_2H_5$ | H | 80–81 |
| O | O | H | 1-Methylcyclopentyl | H | 57–59 |
| O | O | 5-$CF_3$ | Phenyl | H | 133–135 |
| O | O | H | 2,6-Dimethylcyclohexyl | H | 121–123 |
| O | O | 6$OCH_3$ | $CH_3$ | H | 134–136 |
| O | O | H | Cycloheptyl | H | 102–104 |
| O | O | 6$OCH_3$ | Phenyl | H | 209–211 |
| O | S | H | Phenyl | H | |
| O | O | H | Cyclopentyl | H | 110–112 |
| O | O | 6Cl | $CH_3$ | H | 136–138 |

| A | B | X | $R^1$ | $R^2$ | Fp °C |
|---|---|---|---|---|---|
| O | O | H | 3-Methylcyclohexyl | H | 120–122 |
| S | S | H | $CH_3$ | H | |
| S | O | H | $C(CH_3)_3$ | H | |
| S | O | H | $C_2H_5$ | H | |
| S | O | H | Phenyl | H | 156–158 |
| O | O | $C_2H_5$ | Phenyl | H | 56–58 |
| O | O | H | $C(CH_3)_2C_2H_5$ | H | 62–63 |
| O | O | H | $CH(CH_2OCH_3)_2$ | H | 95–96 |
| O | O | H | Cyclohexyl | H | 117–118 |

II. Aminourethane

Beispiel C

Zu einer auf 80°C erwärmten Mischung aus 33 Gew.-Teilen Eisenpulver, 75 Gew.-Teilen Alkohol, 60 Gew.-Teilen Wasser und 3 Gew.-Teilen konz. Salzsäure gibt man unter intensivem Rühren 40 Gew.-Teile 3(S-Methylthiocarbamoyl)-nitro-benzol in solchen Portionen, dass die Temperatur ohne zusätzliche Heizung auf 80°C gehalten wird. Danach kocht man noch 1 Stunde am Rückfluss, saugt heiss ab, digeriert den Rückstand und das Filtrat mit etwa 1000 Gew.-Teilen Methylenchlorid, trocknet über Natriumsulfat, engt ein und kristallisiert aus Toluol um: Fp.: 101–103°C.
Struktur:

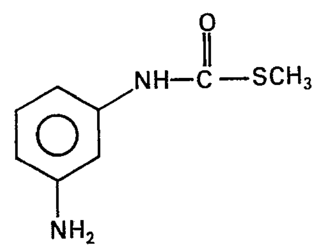

Nach entsprechenden Verfahren können folgende Aminourethane (C) hergestellt werden:

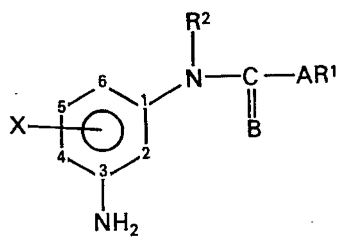

| A | B | X | $R^1$ | $R^2$ | Fp °C |
|---|---|---|---|---|---|
| O | O | H | $CH_3$ | H | 87–89 |
| O | O | $6CH_3$ | $CH_3$ | H | |
| O | O | H | Phenyl | $CH_3$ | 70–72 |
| O | O | 4F | $CH_3$ | H | |
| O | S | H | $CH_3$ | H | |
| S | O | H | $C_2H_5$ | H | |
| O | S | H | Phenyl | H | |
| O | O | $5CF_3$ | $CH_3$ | H | zähes Öl |
| O | O | H | $C_2H_5$ | H | zähes Öl |
| O | O | $2CH_3$ | Phenyl | H | 131–133 |
| O | O | $4CH_3$ | $CH_3$ | H | |
| O | O | 4Cl | Phenyl | H | 215–217 |
| O | O | 4Cl | $CH_3$ | H | |
| O | O | $4CH_3$ | $C_2H_5$ | H | |
| S | S | H | Phenyl | H | |
| O | O | H | 3,3,5-Trimethylcyclohexyl | H | 100–102 |
| O | O | H | Phenyl | $C_2H_5$ | 104–106 |
| O | O | H | Cyclopentyl | H | |
| O | O | $5\text{-}CF_3$ | Phenyl | H | 214–216 |
| O | O | H | Phenyl | H | 178–180 |
| O | O | H | 1-Methylcyclopentyl | H | |
| O | O | H | Hexahydrobenzyl | H | 106–108 |
| O | O | $6OCH_3$ | $CH_3$ | H | 85–87 |

| A | B | X | $R^1$ | $R^2$ | Fp °C |
|---|---|---|---|---|---|
| O | O | H | Cycloheptyl | H | 86–88 |
| O | O | $6OCH_3$ | Phenyl | H | 84–86 |
| O | O | 6Cl | $CH_3$ | H | |
| O | O | H | 3-Methylcyclohexyl | H | 95–97 |
| O | O | H | $CH_2COOCH_3$ | H | zähes Öl |
| S | S | H | $CH_3$ | H | |
| O | O | H | $C(CH_3)_3$ | H | 109–110 |
| O | O | $5\text{-}CF_3$ | $CH(CH_3)_2$ | H | 102–104 |
| O | O | H | $CH_3$ | $CH_3$ | 89–92 |
| O | O | H | 3,5-Dimethylcyclohexyl | H | 80–82 |
| O | O | H | $CH(CH_3)_2$ | H | 66–68 |
| O | O | 4F | $CH_3$ | H | |
| O | O | H | $C(CH_3)_2C_2H_5$ | H | 65–67 |
| O | O | H | Cyclohexyl | H | 122–124 |

III. Nitrohalogenamide und Halogenamidurethane

Beispiel D

Zu 138 Gew.-Teilen 3-Nitroanilin in 1500 Gew.-Teilen Essigsäureäthylester werden 126 Gew.-Teile Natriumhydrogencarbonat gegeben. Unter Rühren tropft man bei 0–10 °C 216 Gew.-Teile 2-Brompropionsäurebromid zu, lässt 16 Stunden bei Raumtemperatur nachrühren, filitriert, engt ein und wäscht die erhaltenen Kristalle mit Toluol: Fp.: 99–101 °C.

Die Verbindung hat folgende Strukturformel (vgl. F)

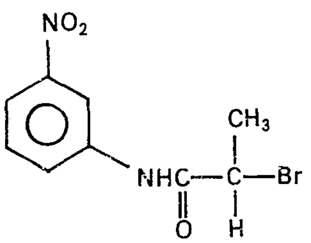

Nach entsprechenden Verfahren können folgende Halogenamidurethane (E) hergestellt werden:

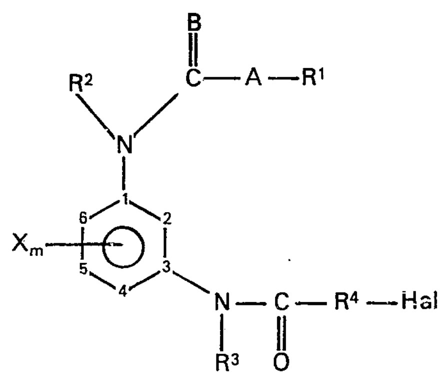

| A | B | X | $R^2$ | $R^1$ | $R^3$ | $R^4$ | Hal | Fp °C |
|---|---|---|---|---|---|---|---|---|
| O | O | H | H | Methyl | H | $-CH(CH_3)-$ | Cl | 138–141 |
| O | O | H | H | Methyl | H | $-CH_2-$ | Cl | 168–170 |
| S | S | H | H | Methyl | H | $-CH(CH_3)-$ | Cl | |
| O | O | H | H | Methyl | H | $-C(CH_3)_2-CH_2-$ | Cl | 125–127 |
| O | O | H | H | Methyl | H | $-CH(C_2H_5)-$ | Br | 131–133 |
| O | O | H | H | Äthyl | H | $-CH_2-$ | Cl | |
| O | O | H | H | Methyl | H | $-C(CH_3)_2-$ | Br | 91–93 |
| O | O | $4CH_3$ | H | Methyl | H | $-CH(CH_3)-$ | Br | 207–210 |
| O | O | H | H | Methyl | H | $-(CH_2)_3-$ | Cl | |
| S | O | H | H | Methyl | H | $-CH(CH_3)-$ | Br | 160–162 |
| O | O | H | H | Methyl | H | $-CH(CH_3)-$ | Br | 142–143 |
| O | S | H | H | Methyl | H | $-CH(CH_3)-$ | Br | |
| O | O | H | H | Phenyl | H | $-CH_2-$ | Cl | 170–173 |
| O | O | H | $CH_3$ | Methyl | H | $-CH(CH_3)-$ | Cl | |
| O | O | H | H | Phenyl | H | $-C(CH_3)_2-CH-$ | Cl | 143–145 |
| O | O | H | H | Phenyl | H | $-CH(CH_3)-$ | Cl | 170–172 |
| O | O | H | H | Methyl | $CH_3$ | $-CH(CH_3)-$ | Br | |

IV. 3-Nitro- und 3-Aminoanilide

Beispiel E

Eine Mischung von 220 Gew.-Teilen d-Brompropionsäure-m-nitroanilid, 206 Gew.-Teilen p-(2,4-Dichlorphenoxy-)phenol, 1000 g Methyläthylketon, 200 g Kaliumcarbonat und 20 g Kaliumjodid wird 10 Stunden unter Rühren am Rückfluss gehalten. Anschliessend wird gekühlt, abgesaugt und das Filtrat eingeengt. Der erhaltene Feststoff wird aus Ethanol umkristallisiert. Fp.: 127—128 °C.

Die Verbindung hat folgende Strukturformel:

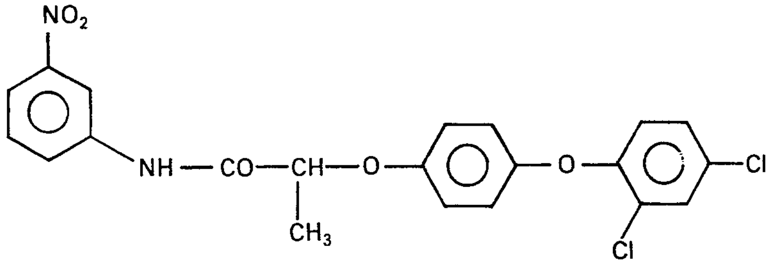

Beispiel F

Eine Mischung aus 295 Gew.-Teilen α-(2,4-Dichlorphenoxyphenoxy-)propionsäure-m-nitro-anilid, 5 Gew.-Teilen 10 %ige Palladium-/Kohlenstoff-Mischung und 1500 Gew.-Teilen Tetrahydrofuran werden bei Raumtemperatur (20 °C) und 100 bar Wasserstoffdruck hydriert. Nach beendeter Wasserstoffaufnahme wird filtriert, das Filtrat eingeengt, in wasserfreiem Ethanol aufgenommen und mit HCL-Gas gesättigt. Anschliessend wird das Hydrochlorid mit Äther ausgefällt und abgesaugt. Schmelzpunkt des Hydrochlorids: 125–128 °C (Zersetzung).

Die Verbindung hat folgende Strukturformel:

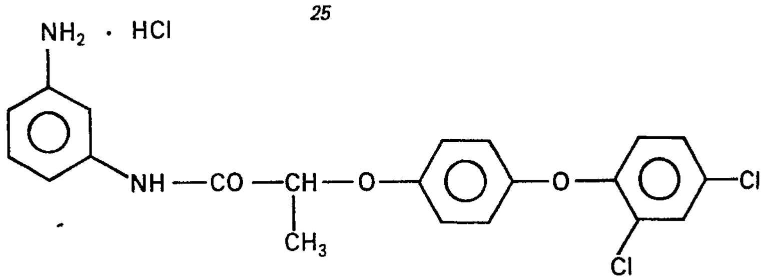

V. m-Anilidurethane

Beispiel 1

13,6 Gew.-Teile α-(2,4-Dichlorphenoxyphenoxy-)propionsäure-m-aminoanilid-hydrochlorid und 6,7 Gew.-Teile Natriumhydrogencarbonat werden in 200 Gew.-Teilen Tetrahydrofuran 30 min. gerührt, anschliessend tropfenweise mit 4,1 Gew.-Teilen Chlorkohlensäure-n-butylester in 30 Gew.-Teilen Tetraphydrofuran versetzt. Nach 2 Stunden wird abgesaugt, das Filtrat eingeengt und über Kieselgel chromatographiert (Laufmittel Methylenchlorid mit 10 % Methanol). Erhalten wird ein hochviskoses Öl, welches nach NMR-Analysendaten die nachstehende Struktur besitzt:

NMR [$CDCl_3$]

| | |
|---|---|
| $>CH-CH_3$ | δ = 1,60 (d) |
| $>CH-CH_3$ | δ = 4,72 (q) |
| $O-CH_2-CH_2-CH_2-CH_3$ | δ = 4,16 (t) |
| $O-CH_2-CH_2-CH_2-CH_3$ | δ = 1,61 (m) |
| $O-CH_2-CH_2-CH_2-CH_3$ | δ = 1,4 (m) |
| $O-CH_2-CH_2-CH_2-CH_3$ | δ = 0,95 (t) |

Analyse für $C_{26}H_{26}Cl_2N_2O_5$ (M = 517)

| | C: | H: | N: | Cl: |
|---|---|---|---|---|
| ber. | 60,36 | 5,07 | 5,41 | 13,70 |
| gef. | 60,6 | 5,2 | 5,2 | 13,3 |

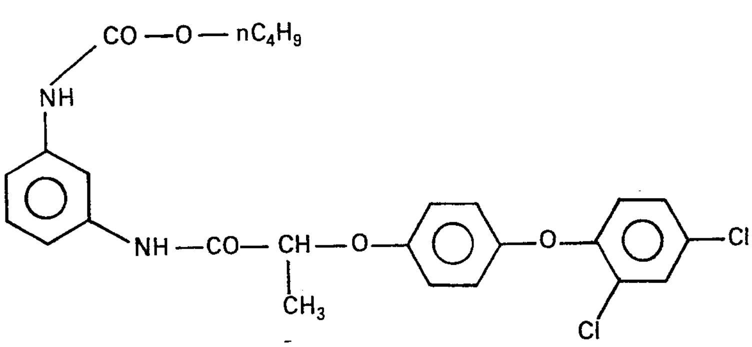

Beispiel 2

Zu einer Mischung von 10,4 Gew.-Teilen 3-(0-tert.-Butylcarbamoyl)-anilin, 5,1 Gew.-Teilen Triäthylamin und 100 Gew.-Teilen Toluol werden bei Raumtemperaturen 19,0 Gew.-Teile α-(2,4-Dichlorphenoxyphenoxy)-propionsäurechlorid in 125 Gew.-Teilen Toluol zugetropft. Nach 3 Stunden wird die Mischung in Eiswasser eingerührt und mit Essigester extrahiert. Die Essigesterphase wird mit Sodalösung ausgewaschen und eingeengt. Es verbleibt amorpher Feststoff, dessen Struktur durch folgende NMR-Daten in $CDCl_3$ sowie durch Elementaranalyse charakterisiert ist:

| | |
|---|---|
| $-C[CH_3]_3$ | $\delta = 1{,}49$ (s) |
| $>CH-CH_3$ | $\delta = 1{,}58$ (d) |
| $>CH-CH_3$ | $\delta = 4{,}58$ (qu) |

Analyse für $C_{26}H_{26}N_2O_5Cl_2$ (M = 517)

| | C | H | Cl | N |
|---|---|---|---|---|
| ber. | 60,36 | 5,07 | 13,70 | 5,41 |
| gef. | 60,8 | 5,4 | 13,3 | 5,1 |

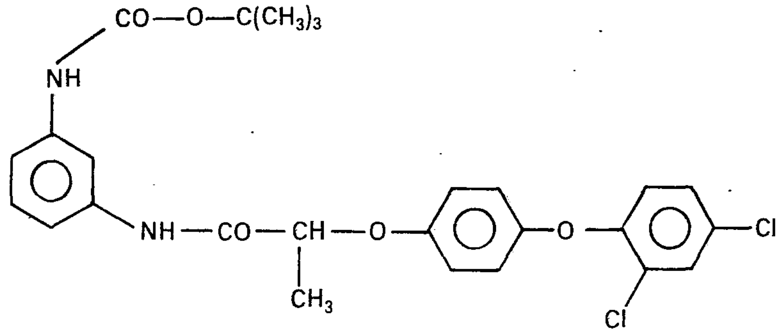

Beispiel 3

16,3 Gew.-Teile α-(2,4-Dichlorphenoxyphenoxy)-propionsäure werden in 200 Gew.-Teilen Tetrahydrofuran gelöst und mit 13 Gew.-Teilen Dicyclohexylcarbodiimid versetzt. Man rührt 2 Stunden bei Raumtemperatur, tropft dann 8,4 Gew.-Teile 3-(0-Methylcarbamoyl)-anilin in 80 Gew.-Teile Tetrahydrofuran hinzu. Nach 15stündigem Rühren wird abgesaugt, das Filtrat eingeengt und der dabei verbleibende ölige Rückstand durch Säulenchromatographie (Laufmittel, Methylenchlorid + 5% Methanol) gereinigt.

Es wird ein Feststoff mit Schmelzpunkt 135°C und nachstehender Struktur erhalten:

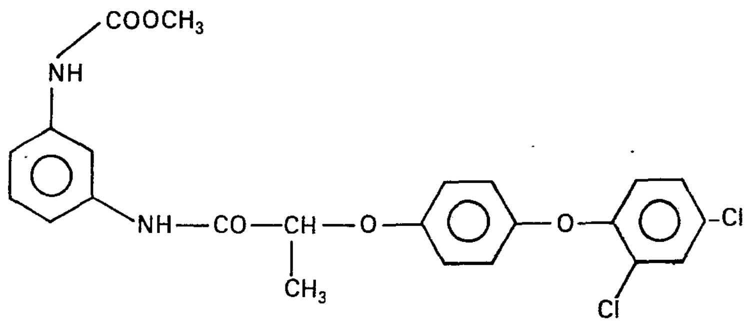

Beispiel 4

16,4 Gew.-Teile α-Brompropionsäure-3-(0-methylcarbamoyl)-anilid werden mit 30 Gew.-Teilen Kaliumcarbonat, 1 Gew.-Teil Kaliumjodid und 14,5 Gew.-Teilen 4(2'-Chlor-4'-nitrophenoxy)-phenol in 200-Gew.-Teilen Methyläthylketon 8 Stunden zum Sieden erhitzt. Nach dem Filtrieren wird eingeengt, der Rückstnd in Methylenchlorid aufgenommen und die Lösung mit verdünnter Natronlauge ausgeschüttelt. Nach dem Trocknen über Natriumsulfat wird das Lösungsmittel abdestilliert und der Rückstand aus einem Toluol-Cyclohexangemisch umkristallisiert.

Man erhält weisse Kristalle vom Fp.: 145–147°C mit folgender Strukturformel:

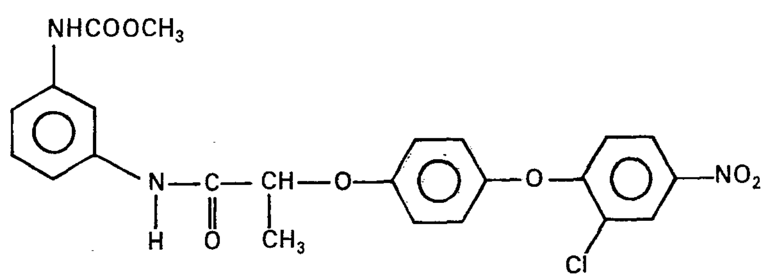

In entsprechender Weise können die folgenden Verbindungen hergestellt werden:

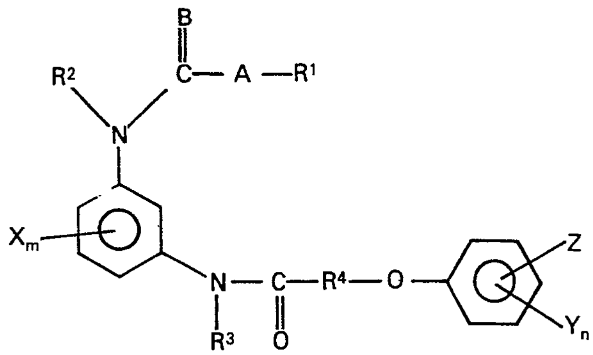

15

| Nr. | A | B | $R^1$ | $R^2$ | $R^3$ | $R^4$ | X | Y | Z | Fp °C |
|---|---|---|---|---|---|---|---|---|---|---|
| 5 | O | O | $CH_3$ | H | H | $-CH(CH_3)-$ | H | H | 4-phenoxy | |
| 6 | O | O | $C_2H_5$ | H | H | $-CH(CH_3)-$ | H | H | 4-(2′,4′-dichlorphenoxy) | 99 |
| 7 | S | S | $CH_3$ | H | H | $-CH(CH_3)-$ | H | H | 4-(2′,4′-dichlorphenoxy) | hochviskos |
| 8 | O | O | $CH_3$ | H | H | $-CH_2-$ | H | H | 4-(4′-[trifluormethyl]phenoxy) | |
| 9 | O | O | $i-C_3H_7$ | H | H | $-CH(CH_3)-$ | H | H | 4-(2′,4′-dichlorphenoxy) | 125 |
| 10 | S | O | $CH_3$ | H | H | $-CH_2-$ | H | H | 4-(2′-nitro-4′-chlorphenoxy) | 166 |
| 11 | O | O | $CH_2CH_2Cl$ | H | H | $-(CH_2)_3-$ | H | 3-Cl | 4-(3′-thiocyanatophenoxy) | |
| 12 | O | O | $cycloC_7H_{13}$ | H | H | $-CH(CH_3)-$ | H | H | 4-(2′,4′-dichlorphenoxy) | hochviskos |
| 13 | O | S | $CH_3$ | H | H | $-CH(CH_3)-$ | H | H | 4-(2′,4′-dichlorphenoxy) | |
| 14 | O | O | $CH_2C{\equiv}CH$ | H | H | $-CH(CH_3)-$ | 4F | H | 4-(2′-methyl-4′-chlorphenoxy) | |
| 15 | O | O | $C_6H_5$ | H | H | $-CH(CH_3)-$ | H | H | 4-(2′,4′-dichlorphenoxy) | 173 |
| 16 | O | O | $CH_2CH{=}CH_2$ | H | H | $-C(C_2H_5)_2-$ | H | H | 4-(3′[methoxycarbonyl]phenoxy) | |
| 17 | S | O | $i-C_3H_7$ | H | H | $-CH(CH_3)-$ | H | H | 4-(2′,4′-dichlorphenoxy) | hochviskos |
| 18 | O | O | $CH_2CH_2CN$ | H | H | $-CH(nC_3H_7)-$ | 6Cl | H | 4-(2′-brom-4′-chlorphenoxy) | |
| 19 | O | O | $CH_3$ | H | H | $-CH_2-$ | H | H | 4-(2′-nitro-4′-chlorphenoxy) | 169 |
| 20 | O | O | $C_2H_5$ | H | H | $-CH(iC_3H_7)-$ | H | 2Br | 4-(2′,4′-dijodphenoxy) | |
| 21 | O | O | $CH_3$ | $CH_3$ | H | $-CH(CH_3)-$ | H | H | 4-(2′,4′-dichlorphenoxy) | 179 |
| 22 | O | O | $CH_2CN$ | $CH_2OCH_3$ | $CH_2OCH_3$ | $CH(C_2H_5H)-$ | H | H | 4-(4′-cyclohexylphenoxy) | |
| 23 | S | O | $CH_3$ | H | H | $-CH_2-$ | $5-CH_3$ | H | 4(4′[thiomethyl]-phenoxy) | |
| 24 | S | O | $C_2H_5$ | H | H | $-CH(C_2H_5)-$ | H | H | 4(4′[dimethylamino]phenoxy) | |
| 25 | S | S | $CH_3$ | H | H | $-CH(CH_2OCH_3)-$ | H | H | 3(3′-methylsulfonyl]phenoxy) | |
| 26 | O | O | $i-C_3H_7$ | H | H | $-C(CH_3)_2-$ | H | H | 4(3′[methoxymethyl]phenoxy) | |
| 27 | O | O | $C_6H_5$ | $C_2H_5$ | H | $-CH(CH_3)-$ | H | H | 4-(2′,4′-dichlorphenoxy) | hochviskos |

| Nr. | A | B | $R^1$ | $R^2$ | $R^3$ | $R^4$ | X | Y | Z | Fp °C |
|---|---|---|---|---|---|---|---|---|---|---|
| 28 | S | O | $t-C_4H_9$ | H | H | $-CH(CH_2Cl)-$ | H | $2-CH_3$ | 4(3'[tetrafluor-äthoxy]phenoxy) | |
| 29 | S | S | $C_2H_5$ | H | H | $-CH_2-$ | H | H | 4(2'-thiocyanato-phenoxy) | |
| 30 | S | O | $i-C_3H_7$ | H | H | $-CH(CH_3)-$ | H | H | 4(2'-[trifluorme-thyl]phenoxy) | |
| 31 | O | O | $CH_3$ | H | H | $-CH(CH_3)-$ | H | H | 4(2'-nitro-4'[tri-fluormethyl]-phenoxy | 134–136 |
| 32 | S | O | $CH_3$ | H | H | $-CH(CH_3)-$ | H | H | 4(2'-chlor-4'[tri-fluormethyl]-phenoxy | 126 |
| 33 | O | O | $CH_3$ | $CH_2Cl$ | H | $-(CH_2)_4-$ | H | $2-NO_2$ | 4(3'[methoxy]-phenoxy) | |
| 34 | O | O | $CH_3$ | H | $i-C_3H_7$ | $-CH(CH_3)CH_2-$ | H | 2-Br | 3(3'[dimethyl-aminosulfonyl]-phenoxy) | |
| 35 | O | O | $CH_3$ | H | $C_2H_5$ | $-CH(CH_3)-$ | $2-CH_3$ | H | 4(4'[acetyl]-phenoxy) | |
| 36 | O | O | $CH(CH_3)CH=CH_2$ | H | H | $-CH(CH_3)CH_2)_2-$ | H | $2-CF_3$ | 4(4'[methyl-aminosulfonyl]-phenoxy) | |
| 37 | O | O | $CH_2C=CCH_2Cl$ | H | H | $-CH(CH_3)-$ | H | 3-Br | 4(2'-formyl-4'-chlorphenoxy) | |
| 38 | O | O | $n-C_3H_7$ | H | H | $-CH(CH_3)-$ | H | $3-CH_3$ | 4(2'-chlor-4'[me-thylsulfonyl]-phenoxy) | |
| 39 | O | O | $CH_3$ | H | H | $-CH(CH_3)-$ | $4-CH_3$ | H | 4(2',4'-dichlor-phenoxy) | 119 |
| 40 | O | O | $CH(CH_3)C\equiv CH$ | H | H | $-CH_2-$ | H | 2-Cl | 4(3'-brom-phenoxy) | |
| 41 | O | O | $t-C_4H_9$ | H | H | $-CH_2$ | $6-OCH_3$ | $3-NO_2$ | 4(4'-fluorphe-noxy) | |
| 42 | O | O | $CH_3$ | H | H | $-CH(CH_3)-$ | H | H | 4(4'-brom-phenoxy) | 130 |
| 43 | O | O | $CH_3$ | H | H | $-CH(CH_3)-$ | $4-OCH_3$ | H | 4(2'-dichlor-phenoxy) | 108 |
| 44 | O | O | $CH(CH_2F)_2$ | H | H | $-CH(CH_3)-$ | H | H | 4(4'-nitropheno-xy) | |
| 45 | O | O | $CH_2CCl=CH_2$ | H | H | $-CH(CH_3)-$ | H | H | 3(3'-nitro-phenoxy) | |
| 46 | O | O | $C_6H_5$ | H | H | $-CH(CH_3)-$ | $5-CF_3$ | H | 4(2',4'-dichlor-phenoxy) | 183 |
| 47 | O | O | $CH_2CH_2OCH_3$ | H | H | $-CH(C_2H_5)-$ | H | H | 4(4'-chlor-phenoxy) | |
| 48 | O | O | cycl.$C_5H_9$ | H | H | $-CH(CH_2OCH_3)-$ | H | H | 3(3'-chlor-phenoxy) | |
| 49 | O | O | $CH_2CH_2F$ | H | H | $-(CH_2)_6-$ | 4-Cl | H | 3(4'-chlor-phenoxy) | |
| 50 | O | O | $CH_2-$ 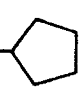 | H | H | $-C(CH_3)_2$ | H | H | 3(2',4'-dichlor-phenoxy | |
| 51 | O | O | $CH_3$ | H | H | $-CH(CH_3)-$ | H | H | 4(4'-fluor-phenoxy) | 121 |
| 52 | O | O | sec.$C_4H_9$ | H | H | $-CH(CH_3)(CH_2)_4-$ | $6-CH_3$ | H | 3(2'-nitro-4'-chlorphenoxy) | |
| 53 | O | O | $CH_3$ | H | H | $-CH(CH_3)-$ | H | H | 4(2'-chlor-4'-bromphenoxy) | 135 |
| 54 | O | O | $t-C_5H_{11}$ | H | H | $-CH(CH_3)-$ | H | H | 3(2',4'-dibrom-phenoxy) | |
| 55 | O | O | 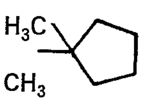 | H | H | $-CH(CH_3)-$ | H | H | 3(3'[trifluorme-thyl]phenoxy) | |
| 56 | O | O | $CH_3$ | H | H | $-CH(CH_3)-$ | H | H | 4(4'-chlorpheno-xy) | 96–97 |

| Nr. | A | B | $R^1$ | $R^2$ | $R^3$ | $R^4$ | X | Y | Z | Fp °C |
|---|---|---|---|---|---|---|---|---|---|---|
| 57 | O | O | $CH_3$ | H | H | $-CH(CH_3)-$ | H | H | 4(2'-nitro-4'-chlorphenoxy) | 152-154 |
| 58 | O | O | $CH_3$ | H | H | $-CH(CH_3)-$ | H | H | 4(4'-nitrophenoxy) | 73-75 |
| 59 | O | O | $CH_3$ | H | H | $-CH(CH_3)-$ | H | H | 3(2'-chlor-4'-nitrophenoxy) | 142 |
| 60 | O | O | $CH_3$ | H | H | $-CH(CH_3)-$ | H | H | 4(2'-chlor-4'[trifluormethyl]-phenoxy) | 103 |
| 61 | O | O | $i-C_3H_7$ | H | H | $-CH(CH_3)-$ | H | H | 4(4']trifluormethyl]phenoxy) | |
| 62 | O | O | $C_2H_5$ | H | H | $-CH(CH_3)-$ | H | H | 4(4'[trifluormethyl]phenoxy) | |
| 63 | O | O | $CH_3$ | H | H | $-CH(CH_3)-$ | H | H | 4(4'[trifluormethyl]phenoxy) | 107-110 |
| 64 | S | O | $CH_3$ | H | H | $-CH(CH_3)-$ | H | H | 4(4'[trifluormethyl]phenoxy) | 137-139 |
| 65 | S | O | $CH_3$ | H | H | $-CH(CH_3)-$ | H | H | 4(2'-brom-4'-[trifluormethyl]-phenoxy) | |
| 66 | O | O | $CH_3$ | H | H | $-CH(CH_3)-$ | H | H | 4(2'[5'-chlorpyridyloxy]) | 98-101 |
| 67 | S | O | $CH_3$ | H | H | $-CH(CH_3)-$ | H | H | 4(2'[5'-jodpyridyloxy]) | |
| 68 | S | S | $CH_3$ | H | H | $-CH(CH_3)-$ | H | H | 4(2'[3',5',6'-trichlorpyridyloxy]) | |
| 69 | O | O | $CH_3$ | H | H | $-CH(CH_3)-$ | H | H | 4(2'[5'-brompyridyloxy]) | |
| 70 | O | O | $CH_3$ | H | H | $-CH(CH_3)-$ | H | H | 4(2'[3',5'-dichlorpyridyloxy]) | |
| 71 | O | O | $CH_3$ | H | H | $-CH(CH_3)-$ | H | H | 4(4'[3'-chlorpyridyloxy]) | |
| 72 | S | O | $CH_3$ | H | H | $-CH(CH_3)-$ | H | H | 4(2'[5'-chlorpyridyloxy]) | |
| 73 | O | O | $C_2H_5$ | H | H | $-CH(CH_3)-$ | H | H | 4(2'[5'-chlorpyridyloxy]) | |
| 74 | O | O | $t-C_4H_9$ | H | H | $-CH(CH_3)-$ | H | H | 4(2'[5'-chlorpyridyloxy]) | |
| 75 | S | O | $CH_3$ | H | H | $-CH(CH_3)-$ | H | H | 4(2'-nitro-4'[trifluormethyl] | 144-146 |
| 76 | O | O | $i-C_3H_7$ | H | H | $CH_2$ | H | H | 4(2'-Nitro-4'-chlorphenoxy) | 137-140 |
| 77 | O | O | $i-C_3H_7$ | H | H | $-CH(CH_3)-$ | H | H | 4(2'-Chlor-4'-trifluormethyl-phenoxy) | 88-90 |
| 78 | S | O | $CH_3$ | H | H | $-CH(CH_3)-$ | H | H | 4(4'-Chlorphenoxy) | hochviskos |
| 79 | S | O | $CH_3$ | H | H | $-CH(CH_3)-$ | H | H | 4(2'-Chlor-4'-bromphenoxy) | 142-146 |
| 80 | S | O | $CH_3$ | H | H | $-CH(CH_3)-$ | H | H | 4(2',4'-Dichlorphenoxy) | 111-113 |

Die neuen Wirkstoffe können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwekken, sie sollen in jedem Fall eine feine und gleichmässige Verteilung der wirksamen Substanzen gewährleisten. Die Formulierungen werden in bekannter Weise hergestellt, z.B. durch Verstrekken des Wirkstoffs mit Lösungsmitteln und/oder Trägerstoffen, gegebenenfalls unter Verwendung von Emulgiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage:

Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Äthanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyäthylen – Fettalkohol – Äther, Alkylsulfonate und Arylsulfonate) und Dispergiermittel, wie Lignin-, Sulfitablaugen und Methylcellulose.

Die herbiziden Mittel enthalten im allgemeinen zwischen 0,1 und 95 Gew.-% Wirkstoff, vorzugsweise zwischen 0,5 und 90.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verspritzen, Verstäuben, Verstreuen oder Giessen.

Die Aufwandmengen liegen je nach Zusammensetzung und Wachstumsstadien der Unkrautflora zwischen 0,1 und 15, vorzugsweise jedoch zwischen 0,2 und 3,0 kg Wirkstoff pro Hektar.

Die neuen herbiziden m-Anilildurethane können unter sich und mit zahlreichen Vertretern anderer herbizider oder wachstumsregulierender Wirkstoffgruppen gemischt und gemeinsam ausgebracht werden. Beispielsweise kommen als Mischungskomponente Diazine, Benzothiadiazinone, 2,6-Dinitroaniline, N-Phenyl-carbamate, Biscarbamate, Thiolcarbamate, Halogencarbonsäuren, Triazine, Amide, Harnstoffe, Diphenyläther, Triazinone, Uracile, Benzofuranderivate und andere in Betracht. Solche Kombinationen dienen zur Verbreitung des Wirkungsspektrums und erzielen zuweilen synergistische Effekte. Beispielsweise können Mischungen mit folgenden Verbindungen hergestellt werden:

5-Amino-4-chlor-2-phenyl-3(2H)-pyridazinon
5-Amino-4-brom-2-phenyl-3-(2H)-pyridazinon
5-Amino-4-chlor-2-cyclohexyl-3-(2H)-pyridazinon
5-Amino-4-brom-2-cyclohexyl-3(2H)-pyridazinon

5-Methylamino-4-chlor-2-m.trifluormethylphenyl-3(2H)-pyridazinon
5-Methylamino-4-chlor-2-m.α,α,β,β-tetrafluoräthoxyphenyl-3(2H)-pyridazinon
5-Dimethylamino-4-chlor-2-phenyl-3(2H)-pyridazinon
4,5-Dimethoxy-2-phenyl-3(2H)-pyridazinon
4,5-Dimethoxy-2-m.trifluormethylphenyl-3(2H)-pyridazinon
5-Methoxy-4-chlor-2-m.trifluormethylphenyl-3(2H)-pyridazinon
5-Amino-4-brom-2-m.methylphenyl-3(2H)-pyridazinon

3-(1-Methyläthyl)-1H-2,1,3-benzothiadiazin-4(3H)-on-2,2-dioxid und Salze
3-(1-Methyläthyl)-8-chlor-1H-2,1,3-benzothiadiazin-4(3H)-on-2,2-dixoid und Salze
3-(1-Methyläthyl)8-fluor-1H-2,1,3-benzothiadiazin-4(3H)-on-2,2-dioxid und Salze
3-(10-Methyläthyl)-8-methyl-1H-2,1,3-benzothiadiazin-4(3H)-on-2,2-dioxid und Salze

1-Methoxymethyl-3-(1-methyläthyl)-2,1,3-benzothiadiazin-4(3H)-on-2,2-dioxid
1-Methoxymethyl-8-chlor-3-(1-methyläthyl)-2,1,3-benzothiadiazin-4(3H)-on-2,2-dioxid
1-Methoxymethyl-8-fluor-3-(1-methyläthyl)-2,1,3-benzothiadiazin-4(3H)-on-2,2-dioxid
1-Cyan-8-chlor-3-(1-methyläthyl)-2,1,3-benzothiadiazin-4(3H)-on-2,2-dioxid
1-Cyan-8-fluor-3-(1-methyläthyl)-2,1,3-benzothiadiazin-4(3H)-on-2,2-dioxid
1-Cyan-8-methyl-3-(1-methyläthyl)-2,1,3-benzothiadiazin-4(3H)-on-2,2-dioxid
1-Cyan-3-(1-methyläthyl)-2,1,3-benzothiadiazin-4(3H)-on-2,2-dioxid
1-Azidomethyl-3-(1-methyläthyl)-2,1,3-benzothiadiazin-4(3H)-on-2,2-dioxid
3-(1-methyläthyl)-1H-(pyridino-[3,2-e]2,1,3-thiadiazin-(4)-on-2,2-dioxid

N-(1-Äthylpropyl)-2,6-dinitro-3,4-dimethylanilin
N-(1-Methyläthyl)-N-äthyl-2,6-dinitro-4-trifluormethyl-anilin
N-n.Propyl-N-β-chloräthyl-2,6-dinitro-4-trifluormethyl-anilin
N-n.Propyl-N-cyclopropylmethyl-2,6-dinitro-4-trifluormethyl-anilin
N-Bis(n.propyl)-2,6-dinitro-3-amino-4-trifluormethylanilin
N-Bis(n.propyl)-2,6-dinitro-4-methyl-anilin
N-Bis(n.propyl)-2,6-dinitro-4-methylsulfonylanilin
N-bis(n.propyl)-2,6-dinitro-4-aminosulfonylanilin
Bis(β-chloräthyl)-2,6-dinitro-4-methyl-anilin
N-Äthyl-N-(2-methylallyl)-2,6-dinitro-4-trifluormethyl-anilin

N-Methylcarbaminsäure-3,4-dichlorbenzylester
N-Methylcarbaminsäure-2,6-di(tert.butyl)-4-methylphenyl-ester
N-Phenylcarbaminsäure-isopropylester
N-3-Fluorphenylcarbaminsäure-3-methoxypropyl-2-ester
N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-1-ester
N-3,4-Dichlorphenylcarbaminsäure-methylester
N-(4-Amino-benzolsulfonyl)-carbaminsäuremethylester
O-(N-Phenylcarbamoyl)-propanonoxim
N-Äthyl-2-(phenylcarbamoyl)-oxypropionsäureamid
3'-N-Isopropyl-carbamoyloxy-propionanilid

Äthyl-N-(3-(N'-phenylcarbamoyloxy)-phenyl)-carbamat
Methyl-N-(3-(N'-methyl-N'-phenylcarbamoyl-oxy)-phenyl)-carbamat
Isopropyl-N-(3-(N'-äthyl-N'-phenylcarbamoyl-oxy)-phenyl)-carbamat
Methyl-N-(3-(N'-3-methylphenylcarbamoyloxy)-phenyl)-carbamat
Methyl-N-(3-(N'-4-fluorphenylcarbamoyloxy)-phenyl)-carbamat
Methyl-N-(3-(N'-3-chlor-4-fluorphenylcarba-moyloxy)-phenyl)-carbamat
Äthyl-N-(3-N'-3-chlor-4-fluorphenylcarba-moyloxy)-phenyl)-carbamat
Äthyl-N-(3-N'-3,4-difluorphenylcarba-moyloxy)-phenyl)-carbamat
Methyl-N-(3-(N'-3,4-difluorphenylcarba-moyloxy)-phenyl)-carbamat

N-3-(4-Fluorphenoxycarbonylamino)-phenylcarbaminsäure-methylester
N-3-(2-Methylphenoxycarbonylamino)-phenylcarbaminsäure-äthylester
N-3-(4-Fluorphenoxycarbonylamino)-phenyl-thiolcarbaminsäure-methylester
N-3-(2,4,5-Trimethylphenoxycarbonylamino)-phenylthiolcarbaminsäure-methylester
N-3-(Phenoxycarbonylamino)-phenylthiol-carbaminsäure-methylester

N,N-Diäthyl-thiolcarbaminsäure-p-chlor-benzylester
N,N-Di n.propyl-thiolcarbaminsäure-äthylester
N,N-Di n.propyl-thiolcarbaminsäure-n.propyl-ester
N,N-Di-isopropyl-thiolcarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolyl-methylester
N,N-Di-isopropyl-thiolcarbaminsäure-3-äthyl-5-isoxazolyl-methylester
N,N-Di-sec.butyl-thiolcarbaminsäure-äthylester
N,N-Di-sec.butyl-thiolcarbaminsäure-benzylester
N-Äthyl-N-cyclohexyl-thiolcarbaminsäure-äthylester
N-Äthyl-N-bicyclo-[2,2,1]-heptyl-thiolcarbamin-säureäthylester

S-(2,3-Dichlorallyl)-(2,2,4-trimetyl-azetidin)-1-carbothiolat
S-(2,3,3-Trichlorallyl)-(2,2,4-trimethyl-azetidin)-1-carbothiolat
S-Äthyl-hexahydro-1-H-azepin-1-carbothiolat
S-Benzyl-3-methylhexahydro-1-H-azepin-1-carbothiolat
S-Benzyl-2,3-dimethylhexahydro-1-H-azepin-1-carbothiolat
S-Äthyl-3-methylhexahydro-1-H-azepin-1-carbothiolat

N-Äthyl-n-n.buthyl-thiolcarbaminsäure-n.propylester

N,N-Dimethyl-dithiocarbaminsäure-2-chlorallylester
N-Methyl-dithiocarbaminsäure-Natrium
Trichloressigsäure-Na salz
α,α-Dichlorpropionsäure-Na salz
α,α-Dichlorbuttersäure-Na salz
α,α,β,β-Tetrafluorpropions äure-Na salz
α-Chlor-β-(4-chlorphenyl)-propionsäure-methylester
α,β-Dichlor-β-phenylpropionsäure-methylester
Benzamido-oxy-essigsäure
2,3,5-Trijodbenzoesäure (Salze, Ester, Amide)
2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)
O,S-Dimethyl-tetrachlor-thioterephtalat
Dimethyl-2,3,5,6-tetrachlor-terephthalat
Dinatrium-3,6-endoxohexahydro-phthalat
4-Amino-3,5,6-trichlor-picolinsäure (Salze)
2-Cyan-3-(N-methyl-N-phenyl)-amino-acrylsäureäthylester
2-[4-(4'-Chlorphenoxy)-phenoxy]-propionsäure-isobutylester
2-[4-(2',4'-Dichlorphenoxy)-phenoxy]-propion-säuremethylester
2-[4-(4'-Trifluormethylphenoxy)-phenoxy]-propionsäure-methylester
2-[4-(2'-Chlor-4'-trifluorphenoxy)-phenoxy]-propionsäure-Na salz
2-[4-(3',5'-Dichlorpyridyl-2-oxy)-phenoxy]-propionsäure-Na salz

2-(N-Benzoyl-3,4-dichlorphenylamino)-propionsäureäthylester
2-(N-Benzoyl-3-chlor-4-fluorphenylamino)-propionsäure-methylester
2-(N-Benzoyl-3-chlor-4-fluorphenylamino)-propionsäureisopropylester
2-Chlor-4-äthylamino-6-isopropylamino-1,3,5-triazin
2-Chlor-4-äthylamino-6-(amino-2'-propionitril)-1,3,5-triazin
2-Chlor-4-äthylamino-6-2-methoxypropyl-2-amino-1,3,5-triazin
2-Chlor-4-äthylamino-6-butin-1-yl-2-amino-1,3,5-triazin
2-Chlor-4,6-bisäthylamino-1,3,5-triazin
2-Chlor-4,6-bisisopropylamino-1,3,5-triazin
2-Chlor-4-isopropylamino-6-cyclopropyl-amino-1,3,5-triazin
2-Azido-4-methylamino-6-isopropylamino-1,3,5-triazin
2-Methylthio-4-äthylamino-6-isopropyl-amino-1,3,5-triazin
2-Methylthio-4-äthylamino-6-tert.butylami-no-1,3,5-triazin
2-Methylthio-4,6-bisäthylamino-1,3,5-triazin
2-Methylthio-4,6-bisisopropylamino-1,3,5-triazin
2-Methoxy-4-äthylamino-6-isopropylamino-1,3,5-triazin
2-Methoxy-4,6-bisäthylamino-1,3,5-triazin
2-Methoxy-4,6-bisisopropylamino-1,3,5-triazin
4-Amino-6-tert.butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert.butyl-3-methylthio-4,5-dihydro-1,2,4-triazin-5-on
1-Methyl-3-cyclohexyl-6-dimethylamino-1,3,5-triazin-2,4-dion
3-tert.Butyl-5-chlor-6-methyluracil
3-tert.Butyl-5-brom-6-methyluracil
3-Isopropyl-5-brom-6-methyluracil
3-sec.Butyl-5-brom-6-methyluracil
3-(2-Tetrahydropyranyl)-5-chlor-6-methyluracil
3-(2-Tetrahydropyranyl)-5,6-trimethylenuracil
3-Cyclohexyl-5,6-trimethylenuracil
2-Methyl-4-(3'-trifluormethylphenyl)-tetrahydro-1,2,4-oxadiazin-3,5-dion
2-Methyl-4-(4'-fluorphenyl)-tetrahydro-1,2,4-oxadiazin-3,5-dion
3-Amino-1,2,4-triazol
1-Allyloxy-1-(4-bromphenyl)-2-[1',2',4'-triazolyl-(1')-]-äthan (Salze)
[-1-(1,2,4-Triazolyl-1')]-[1(4'-chlorphenoxy)]-3,3-dimethylbutan-2-on
N,N-Diallylchloracetamid
N-Isopropyl-2-chloracetanilid
N-(Butin-1-yl-3)-2-chloracetanilid
2-Methyl-6-äthyl-N-(propargyl)-2-chloracetanilid
2-Methyl-6-äthyl-N-(äthoxymethyl)-2-chloracetanilid
2-Methyl-6-äthyl-N-(2-methoxy-1-methyläthyl)-2-chloracetanilid
2-Methyl-6-äthyl-N-(isopropoxycarbonyläthyl)-2-chloracetanilid
2-Methyl-6-äthyl-N-(4-methoxypyrazol-1-ylmethyl)-2-chloracetanilid
2-Methyl-6-äthyl-N-(pyrazol-1-yl-methyl)-2-chloracetanilid
2,6-Dimethyl-N-(pyrazol-1-yl-methyl)-2-chloracetanilid
2,6-Dimethyl-N-(4-methylpyrazol-1-ylmethyl)-2-chlor-acetatanilid
2,6-Dimethyl-N-(1,2,4-triazol-1-yl-methyl)-2-chloracetanilid
2,6-Dimethyl-N-(3,5-dimethylpyrazol-1-ylmethyl)-2-chloracetanilid
2,6-Dimethyl-N-(1,3-dioxalan-2-yl-2-ylmethyl)-2-chloracetanilid
2,6-Dimethyl-N-(2-methoxyäthyl)-2-chloracetanilid
2,6-Dimethyl-N-(isobutoxymethyl)-2-chloracetanilid
2,6-Diäthyl-N-(methoxymethyl)-2-chloracetanilid
2,6-Diäthyl-N-(n.butoxymethyl)-2-chloracetanilid
2,6-Diäthyl-N-(äthoxycarbonylmethyl)-2-chloracetanilid
2,3,6-Trimethyl-N-(pyrazol-1-yl-methyl)-2-chloracetanilid
2,3-Dimethyl-N-(isopropyl)-2-chloracetanilid
2-(2-Methyl-4-chlorphenoxy-4-N-methoxyacetamid
2-(α-Naphthoxy)-N,N-diäthylpropionamid
2,2-Diphenyl-N,N-dimethylacetamid
α(3,4,5-Tribrompyrazol-1-yl)-N,N-dimethylpropionamid
N-(1,1-Dimethylpropinyl)-3,5-dichlorbenzamid
N-1-Naphthylphthalamidsäure
Propionsäure-3,4-dichloranilid
Cyclopropancarbonsäure-3,4-dichloranilid
Methacrylsäure-3,4-dichloranilid
2-Methylpentancarbonsäure-3,4-dichloranilid
N-2,4-Dimethyl-5-(trifluormethyl)-sulfonylamino-phenylacetamid
2-Propionyl-amino-4-methyl-5-chlor-thiazol
O-(Methylsulfonyl)-glykolsäure-N-äthoxymethyl-2,6-dimethylanilid
O-(Methylaminosulfonyl)-glykolsäure-N-isopropyl-anilid
O-(i-Propylaminosulfonyl)-glykolsäure-N-butin-1-yl-3-anilid
O-(Methylaminosulfonyl)-glykolsäure-hexamethylenimid
2,6-Dichlor-thiobenzamid
2,6-Dichlorbenzonitril
3,5-Dibrom-4-hydroxy-benzonitril (Salze)
3,5-Dijod-4-hydroxy-benzonitril (Salze)
3,5-Dibrom-4-hydroxy-O-2,4-dinitrophenylbenzaldoxim (Salze)
3,5-Dibrom-4-hydroxy-O-2-Cyan-4-nitrophenylbenzaldoxim (Salze)
Pentachlorphenol-Natriumsalz
2,4-Dichlorphenyl-4'-nitrophenyläther
2,4,6-Trichlorphenyl-4'-nitrophenyläther
2-Fluor-4,6-dichlorphenyl-4'-nitrophenyläther
2-Chlor-4-trifluormethylphenyl-4'-nitrophenyläther
2,4'-Dinitro-4-trifluormethyl-diphenyläther
2,4-Dichlorphenyl-3'-methoxy-4'-nitro-phenyläther
2-Chlor-4-trifluormethylphenyl-3'-äthoxy-4'-nitro-phenyläther
2-Chlor-4-trifluormethylphenyl-3'-carboxy-4'-nitro-phenyläther (Salze)
2,4-Dichlorphenyl-3'-methoxycarbonyl-4'-nitrophenyläther
2-(3,4-Dichlorphenyl)-4-methyl-1,2,4-oxadiazolidin-3,5-dion
2-(3-tert.Butylcarbamoyl-oxyphenyl)-4-methyl-1,2,4-oxadiazolidin-3,5-dion
2-(3-iso.Propylcarbamoyl-oxyphenyl)-4-methyl-1,2,4-oxadiazolidin-3,5-dion
2-Phenyl-3,1-benzoxazinon-(4)
(4-Bromphenyl)-3,4,5,9,10-pentaazatetracyclo-[5,4,1,$0^{2,6}$,$0^{8,11}$]-dodeca-3,9-dien
2-Äthoxy-2,3-dihydro-3,3-dimethyl-5-benzofuranyl-methan-sulfonat
2-Äthoxy-2,3-dihydro-3,3-dimethyl-5-benzofuranyl-dimethyl-aminosulfonat
2-Äthoxy-2,3-dihydro-3,3-dimethyl-5-benzofuranyl-(N-methyl-N-acetyl)-aminosulfonat
3,4-Dichlor-1,2-benzisothiazol
N-4-Chlorphenyl-allylbernsteinsäureimid
2-Methyl-4,6-dinitrophenol (Salze, Ester)

2-sec.Butyl-4,6-dinitrophenol (Salze, Ester)
2-sec.Butyl-4,6-dinitrophenol-acetat
2-tert.Butyl-4,6-dinitrophenol-acetat
2-tert.Butyl-4,6-dinitrophenol (Salze)
2-tert.Butyl-5-methyl-4,6-dinitrophenol (Salze)
2-tert.Butyl-5-methyl-4,6-dinitrophenol-acetat

2-sec.Amyl-4,6-dinitrophenol (Salze, Ester)
1-(α,α-Dimethylbenzyl)-3-(4-methylphenyl)-harnstoff
1-Phenyl-3-(2-methylcyclohexyl)-harnstoff
1-Phenyl-1-benzoyl-3,3-dimethyl-harnstoff
1-(4-chlorphenyl)-1-benzoyl-3,3-dimethyl-harnstoff
1-(4-chlorphenyl)-3,3-dimethyl-harnstoff
1-(4-Chlorphenyl)-3-methyl-3-butin-1-yl-3-harnstoff
1-(3,4-Dichlorphenyl)-3,3-dimethyl-harnstoff
1-(3,4-Dichlorphenyl)-1-benzoyl-3,3-dimethyl-harnstoff
1-(3,4-Dichlorphenyl)-3-methyl-3-n.butyl-harnstoff
1-(4-i-Propylphenyl)-3,3-dimethyl-harnstoff
1-(3-Trifluormethylphenyl)-3,3-dimethyl-harnstoff
1-(3-α,α,β,β-Tetrafluoräthoxyphenyl)-3,3-dimethyl-harnstoff
1-(3-tert.Butylcarbamoyloxy-phenyl)-3,3-dimethyl-harnstoff
1-(3-Chlor-4-methylphenyl)-3,3-dimethyl-harnstoff
1-(3-Chlor-4-methoxyphenyl)-3,3-dimethyl-harnstoff
1-(3,5-Dichlor-4-methoxyphenyl)-3,3-dimethyl-harnstoff
1-[4(4'-Chlorphenoxy)-phenyl]-3,3-dimethyl-harnstoff
1-[4(4'-methoxyphenoxy)-phenyl]-3,3-dimethyl-harnstoff
1-Cyclooktyl-3,3-dimethyl-harnstoff
1-(Hexahydro-4,7-methanindan-5-yl)-3,3-dimethyl-harnstoff
1-[1- oder 2-(3a,4,5,7,7a-Hexahydro)-4,7-methanoindanyl]-3,3-dimethyl-harnstoff
1-(4-Fluorphenyl)-3-carboxymethoxy-3-methyl-harnstoff
1-Phenyl-3-methyl-3-methoxy-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-methoxy-harnstoff
1-(3-Chlor-4-bromphenyl)-3-methyl-3-methoxy-harnstoff
1-(3-Chlor-4-isopropylphenyl)-3-methyl-3-methoxy-harnstoff
1-(3-Chlor-4-methoxyphenyl)-3-methyl-3-methoxy-harnstoff
1-(3-tert.Butylphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethyl-harnstoff
Imidazolidin-2-on-1-carbonsäure-iso-butylamid
1,2-Dimethyl-3,5-diphenylpyrazolium-methylsulfat
1,2,4-Trimethyl-3,5-diphenylpyrazolium-methylsulfat
1,2-Dimethyl-4-brom-3,5-diphenylpyrazolium-methylsulfat
1,3-Dimethyl-4-(3,4-dichlorbenzoyl)-5-[(4-methylphenyl-sulfonyl)-oxy]-pyrazol
2,3,5-Trichlor-pyridinol-(4)
1-Methyl-3-phenyl-5-(3'-trifluormethylphenyl)-pyridon-(4)

1-Methyl-4-phenyl-pyridiniumchlorid
1,1-Dimethylpyridiniumchlorid
3-Phenyl-4-hydroxy-6-chlorpyridazin
1,1'-Di(3,5-dimethylmorpholin-carbonylmethyl)-4,4'-di-pyridylium-dichlorid
1,1'-Äthylen-2,2'-dipyridylium-dibromid
3-[1(N-Äthoxyamino)-propyliden]-6-äthyl-3,4-dihydro-2-H-pyran-2,4-dion
3-[1-(N-Allyloxyamino)-propyliden]-6-äthyl-3,4-dihydro-2-H-pyran-2,4-dion
2-[1-(N-Allyloxyamino)-propyliden]-5,5-dimethylcyclohexan-1,3-dion (Salze)
2-[1-(N-Allyloxyamino-butyliden]-5,5-dimethylcyclohexan-1,3-dion (Salze)
2-[1-(N-Allyloxyamino-butyliden]-5,5-dimethyl-4-methoxy-carbonyl-cyclohexan-1,3-dion (Salze)
2-[1-(N-Allyloxyamino-butyliden]-5,5-dimethyl-4-methoxy-carbonyl-cyclohexan-1,3-dion (Salze)
2-Chlorphenoxyessigsäure (Salze, Ester, Amide)
4-Chlorphenoxyessigsäure (Salze, Ester, Amide)
2,4-Dichlorphenoxyessigsäure (Salze, Ester, Amide)
2,4,5-Trichlorphenoxyessigsäure (Salze, Ester, Amide)
2-Methyl-4-chlorphenoxyessigsäure (Salze, Ester, Amide)
3,5,6-Trichlor-2-pyridinyl-oxyessigsäure (Salze, Ester, Amide)

α-Naphthoxyessigsäuremethylester
2-(2-Methylphenoxy)-propionsäure (Salze, Ester, Amide)
2-(4-Chlorphenoxy)-propionsäure (Salze, Ester, Amide)
2-(2,4-Dichlorphenoxy)-propionsäure (Salze, Ester, Amide)
2-(2,4,5-Trichlorphenoxy)-propionsäure (Salze, Ester, Amide)
2-(2-Methyl-4-chlorphenoxy)-propionsäure (Salze, Ester, Amide)
4-(2,4-Dichlorphenoxy)-buttersäure (Salze, Ester, Amide)
4-(2-Methyl-4-chlorphenoxy)-buttersäure (Salze, Ester, Amide)
Cyclohexyl-3-(2,4-dichlorphenoxy-acrylat
9-Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure (Salze, Ester)
Gibellerinsäure (Salze)
Dinatrium-methylarsonat
Mononatriumsalz der Methylarsonsäure
N-Phosphon-methyl-glycin (Salze)
N,N-Bis(phosphonmethyl)-glycin (Salze)

2-Chloräthanphosphonsäure-2-chloräthylester
Ammonium-äthyl-carbamoyl-phosphonat
Di-n.butyl-1-n.butylamino-cyclohexyl-phosphonat
Trithiobutylphosphit
O,O-Diisopropyl-5-(2-benzosulfonylamino-äthyl)-phosphordithioat
2,3-Dihydro-5,6-dimethyl-1,4-dithiin-1,1,4,4-tetraoxid
5-tert.Butyl-3-(2,4-dichlor-5-isopropoxyphenyl)-1,3,4-oxadiazolon-(2)
4,5-Dichlor-2-trifluormethyl-benzimidazol (Salze)
1,2,3,6-Tetrahydropyridazin-3,6-dion (Salze)
Bernsteinsäure-mono-N-dimethylhydrazid (Salze)
(2-Chloräthyl)-trimethyl-ammoniumchlorid
(2-Methyl-4-phenylsulfonyl)-trifluormethan-sulfonanilid
1,1-Dimethyl-4,6-diisopropyl-5-indanyläthyl-keton
Natriumchlorat
Ammoniumrhodanid
Calciumcyanamid

Ausserdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden.

Zur Aktivierung der herbiziden Wirkung können Netz- und Haftmittel sowie nicht-phytotoxische Öle und Ölkonzentrate zugesetzt werden.

Der Einfluss verschiedener Vertreter der erfindungsgemässen Verbindungen auf das Wachstum von unerwünschten und erwünschten Pflanzen wird in nachfolgenden Gewächshausversuchen demonstriert:

Die Samen der Testpflanzen entsprechend Tabelle 1 wurden nach Arten getrennt flach eingesät. Unmittelbar danach erfolgte bei Vorauflaufbehandlung das Aufbringen der Wirkstoffe auf die Erdoberfläche. Die Wirkstoffe wurden hierbei in Wasser als Verteilungsmittel suspendiert oder emulgiert und mittels fein verteilender Düsen gespritzt. Nach dem Aufbringen der Mittel wurden die Gefässe leicht beregnet, um Keimung und Wachstum in Gang zu bringen und auch gleichzeitig die Wirkstoffe zu aktivieren. Danach deckte man die Gefässe mit durchsichtigen Plastikhauben ab, bis die Pflanzen angewachsen waren. Diese Abdeckung bewirkte ein gleichmässiges Keimen der Testpflanzen, sofern dies nicht durch die Wirkstoffe beeinträchtigt wurde, und verhinderte das Verdampfen leicht flüchtiger Substanzen.

Zum Zwecke der Nachauflaufbehandlung zog man die Pflanzen je nach Wuchsform in den Versuchsgefässen erst bis zu einer Höhe von 3 bis 10 cm an und behandelte sie danach. Eine Abdekkung unterblieb. Die Aufstellung der Versuche erfolgte im Gewächshaus, wobei für wärmeliebende Arten wärmere Bereiche (25 bis 40°C) und für solche gemässigter Klimate 15 bis 30°C bevorzugt wurden. Die Versuchsperiode erstreckte sich über 4 Wochen. Während dieser Zeit wurden die Pflanzen gepflegt und ihre Reaktion auf die einzelnen Behandlungen wurde ausgewertet. Die folgenden Tabellen enthalten die Prüfsubstanzen, die jeweiligen Dosierungen und kg/ha Aktivsubstanz und die Testpflanzenarten. Bewertet wird nach einer Skale von 0 bis 100. Dabei bedeutet 0 keine Schädigung oder normaler Ablauf und 100 kein Aufgang der Pflanzen bzw. völlige Zerstörung zumindest der oberirdischen Sprossteile.

Die beigefügten Tabellen präsentieren die selektive Wirkung von Vertretern der erfindungsgemässen Verbindungen. Diese richtet sich in erster Linie gegen unerwünschte Gräser. Dabei sind die Mittel sowohl für einige monokotyle wie für zahlreiche dikotyle Kulturpflanzen sehr gut verträglich. Sie wurden im Vorauflauf- und Nachauflaufverfahren eingesetzt. Eine besondere Aufbringungstechnik besteht darin, dass die Wirkstoffe mit Hilfe der Spritzgeräte so gespritzt werden, dass die Blätter empfindlicher Kulturpflanzen nach Möglichkeit nicht getroffen werden, während die Wirkstoffe auf die darunterliegende Bodenfläche oder dort wachsende unerwünschte Pflanzen gelangen (post-directed, lay-by).

In Anbetracht der Vielseitigkeit der Applikationsmethoden können die erfindungsgemässen Mittel oder diese enthaltende Mischungen noch in einer weiteren grossen Zahl von Kulturen zur Beseitigung unerwünschten Pflanzenwuchses eingesetzt werden.

Im einzelnen seien folgende Nutzpflanzen genannt:

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuss | peanuts (groundnuts) |
| Asparagus officinalis | Spargel | asparagus |
| Aventa sativa | Hafer | oats |

| Botanischer Name | Deutscher Name | Englischer Name |
|---|---|---|
| Beta vulgaris spp. altissima | Zuckerrübe | sugarbeets |
| Beta vulgaris spp. rapa | Futterrübe | fodder beets |
| Beta vulgaris spp. esculenta | Rote Rübe | table beets, red beets |
| Brassica napus var. napus | Raps | rape seed |
| Brassica napus var. napobrassica | Kohlrübe | |
| Brassica napus var. rapa | Weisse Rübe | turnips |
| Brassica rapa var. silvestris | Rübsen | |
| Camellia sinensis | Teestrauch | tea plants |
| Carthamus tinctorius | Saflor-Färbedistel | safflower |
| Carya illinoinensis | Pekannussbaum | pecan trees |
| Citrus limon | Zitrone | lemon |
| Citrus maxima | Pampelmuse | grapefruits |
| Citrus reticulata | Mandarine | |
| Citrus sinensis | Apfelsine, Orange | orange trees |
| Coffea arabica (Coffea canephora, Coffea liberica) | Kaffee | coffee plants |
| Cucumis melo | Melone | melons |
| Cucumis sativus | Gurke | cucumber |
| Cynodon dactylon | Bermudagras | Bermudagrass in turfs and lawns |
| Daucus carota | Möhre | carrots |
| Elaeis guineensis | Ölpalme | oil palms |
| Fragaria vesca | Erdbeere | strawberries |
| Glycine max | Sojabohne | soybeans |
| Gossypium hirsutum (Gossypium arboreum, Gossypium herbaceum, Gossypium vitifolium) | Baumwolle | cotton |
| Helianthus annuus | Sonnenblume | sunflowers |
| Helianthus tuberosus | Topinambur | |
| Hevea brasiliensis | Parakautschukbaum | rubber plants |
| Hordeum vulgare | Gerste | barley |
| Humulus lupulus | Hopfen | hop |
| Ipomoea batatas | Süsskartoffeln | sweet potato |
| Juglans regia | Walnussbaum | walnut trees |
| Lactua sativa | Kopfsalat | lettuce |
| Lens culinaris | Linse | lentils |
| Linum usitatissimum | Faserlein | flax |
| Lycopersiocon lycopersicum | Tomate | tomato |
| Malus spp. | Apfel | apple trees |
| Manihot esculenta | Maniok | cassava |
| Medicago sativa | Luzerne | alfalfa (lucerne) |
| Mentha piperita | Pfefferminze | peppermint |
| Musa spp. | Obst- u. Mehlbanane | banana plants |
| Nicotiana tabacum (N. rustica) | Tabak | tabacco |
| Olea europaea | Ölbaum | olive trees |
| Oryza sativa | Reis | rice |
| Panicum miliaceum | Rispenhirse | |
| Phaseolus lunatus | Mondbohne | limabeans |
| Phaseolus mungo | Urbohne | mungbeans |
| Phaseolus vulgaris | Buschbohnen | snapbeans, green beans, dry beans |
| Pennisetum glaucum | Perl- oder Rohrkolbenhirse | |
| Petroselinim crispum spp. tuberosum | Wurzelpetersilie | parsley |
| Picea abies | Rotfichte | Norway spruce |
| Abies alba | Weisstanne | fire |
| Pinus spp. | Kiefer | pine trees |
| Pisum sativum | Gartenerbse | English peas |
| Prunus avium | Süsskirsche | cherry trees |
| Prunus domestica | Pflaume | plum trees |
| Prunus dulcis | Mandelbaum | almond trees |
| Prunus persica | Pfirsich | peach trees |
| Pyrus communis | Birne | pear trees |
| Ribes sylvestre | Rote Johannisbeere | red currants |

| Botanischer Name | Deutscher Name | Englischer Name |
|---|---|---|
| Ribes uva-crispa | Stachelbeere | |
| Ricinus communis | Rizinus | |
| Saccharum officinarum | Zuckerrohr | sugar cane |
| Secale cereale | Roggen | rye |
| Sesamum indicum | Sesam | Sesame |
| Solanum tuberosum | Kartoffel | Irish potato |
| Sorghum bicolor (s. vulgare) | Mohrenhirse | sorghum |
| Sorghum dochna | Zuckerhirse | |
| Spinacia oleracea | Spinat | spinach |
| Theobroma cacao | Kakaobaum | cacao plants |
| Trifolium pratense | Rotklee | red clover |
| Triticum aestivum | Weizen | wheat |
| Vaccinium carymbosum | Kulturheidelbeere | blueberry |
| Vaccinium vitis-idaea. | Preisselbeere | cranberry |
| Vicia faba | Pferdebohnen | tick beans |
| Vigna sinensis (V. unguiculata) | Kuhbohne | cow peas |
| Vitis vinifera | Weinrebe | grapes |
| Zea mays | Mais | Indian corn, sweet corn, maize |

Tabelle 1: Liste der Pflanzennamen

| Botanischer Name | Abkürz. in Tabelle | Deutscher Name | Englischer Name |
|---|---|---|---|
| Allium cepa | | Küchenzwiebel (gesät) | onion |
| Alopecurus myosuroides | Alopec. myosur. | Ackerfuchsschwanz | slender foxtail |
| Amaranthus retroflexus | | Zurückgekrümmter Fuchsschwanz | redroot pigweed |
| Arachys hypogaea | | Erdnuss | peanuts (groundnuts) |
| Beta vulgaris | | Zuckerrübe | sugarbeets |
| Bracchiaria spp. | | | signalgrass |
| Brassica napus | | Weisse Rübe | turnips |
| Bromus tectorum | | Dachtrespe | downy broome |
| Echinochloa crus galli | Echinochl. c.g. | Hühnerhirse | barnyardgrass |
| Eleusine indica | | | goosegrass |
| Euphorbia geniculata | | Südamerikanische Wolfsmilchart | southamerican member of the spruge family |
| Glycine max | | Sojabohnen | soybeans |
| Gossypium hirsutum | | Baumwolle | cotton |
| Ipomoea spp. | | Prunkwindearten | morningglory |
| Lolium multiflorum | | Italien. Raygras | Italian ryegrass |
| Lycopersicon esculentum | Lycopersicon esculent. | Tomate | tomato |
| Pisum sativum | | Erbsen | english peas |
| Poa trivialis | | Gemeines Rispengras | bluegrass |
| Sesbania exaltata | | Turibaum | hemp sesbania (coffeeweed) |
| Setaria faberi | | Borstenhirse | grant foxtail |
| Sinapis alba | | Weisser Senf | white mustard |
| Sorghum halepense (aus Samen) | | Sudangras | Johnsongrass |
| Triticum aestivum | | Weizen | wheat |

Tabelle 2:
Selektive Bekämpfung unerwünschter Gräser bei Vorlaufanwendung im Gewächshaus

Wirkstoff Nr. 56

| Testpflanzen | Schädigung [%] bei ca. 1,0 kg/ha |
|---|---|
| Allium cepa | 3 |
| Beta vulgaris | 3 |
| Brassica napus | 3 |
| Glycine max | 0 |
| Gossypium hirsutum | 0 |
| Pisum sativum | 5 |
| Brachiaria spp. | 83 |
| Echinochloa crus galli | 87 |
| Eleusine indica | 100 |
| Poa trivialis | 90 |
| Setaria faberi | 84 |

0 = keine Schädigung
100 = Pflanzen abgestorben

Tabelle 3:
Bekämpfung von Sorghum halepense und anderen unerwünschten Gräsern in breitblättrigen Kulturen bei Nachauflaufanwendung im Gewächshaus

Wirkstoff Nr. 31

| Testpflanzen | Schädigung [%] bei 1,0 kg/ha |
|---|---|
| Arachys hypogaea | 0 |
| Brassica napus | 10 |
| Gossypium hirsutum | 5 |
| Lycopersicon esculent. | 0 |
| Alopec. myosur. | 78 |
| Bromus tectorum | 90 |
| Setaria faberi | 80 |
| Sorghum halepense (aus Samen) | 87 |

0 = keine Schädigung
100 = Pflanzen abgestorben

Tabelle 4:
Selektive Bekämpfung von unerwünschten Grasarten in verschiedenen Kulturen bei Nachauflaufanwendung im Gewächshaus

Wirkstoff Nr. 53

| Testpflanzen | Schädigung [%] bei 1,0 kg/ha |
|---|---|
| Arachys hypogaea | 0 |
| Beta vulgaris | 10 |
| Brassica napus | 0 |
| Glycine max | 10 |
| Gossypium hirsutum | 10 |
| Lycopersicon esculent. | 0 |
| Triticum aestivum | 0 |
| Alopec. myosur. | 80 |
| Echinochloa crus galli | 98 |
| Setaria faberi | 98 |

0 = keine Schädigung
100 = Pflanzen abgestorben

Tabelle 5:
Wirkungsbeispiel eines bekannten Wirkstoffs aus DE-AS 17 93 226 bei Nachauflaufanwendung im Gewächshaus

Wirkstoff

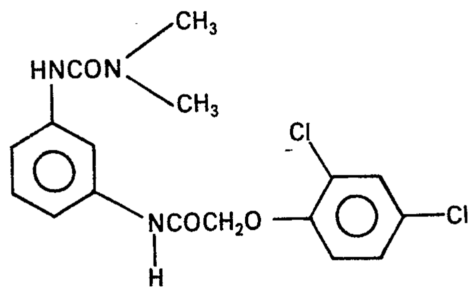

| Testpflanzen | Schädigung [%] bei 1,0 kg/ha |
|---|---|
| Arachys hypogaea | 0 |
| Beta vulgaris | 5 |
| Brassica napus | 30 |
| Glycine max | 32,5 |
| Gossypium hirsutum | 34 |
| Triticum aestivum | 10 |
| Alopec. myosur. | 5 |
| Brachiaria spp. | 0 |
| Echinochloa crus galli | 0 |
| Sorghum halepense (aus Samen) | 0 |
| Amaranthus retroflexus | 10 |
| Euphorbia geniculata | 76 |
| Ipomoea spp. | 75 |
| Sinapis alba | 80 |
| Sesbania exaltata | 95 |

0 = keine Schädigung
100 = Pflanzen abgestorben

Das Beispiel zeigt eine starke Schädigung der Nutzpflanzen durch den bekannten Wirkstoff, dessen Wirksamkeit sich auf breitblättrige Pflanzen beschränkt

Tabelle 6:

Herbizide Wirkung weiterer Verbindungen bei Vor- und Nachauflaufanwendung im Gewächshaus

| Wirkstoff Nummer | kg/ha | Testpflanzen und [%] Schädigung Vorauflauf Lolium m. | Echinochl. c.g. | Nachauflauf Lolium m. | Echinochl. c.g. |
|---|---|---|---|---|---|
| 3 | 3 | 100 | 100 | 100 | 90 |
| 6 | 3 | 90 | 90 | 80 | 90 |
| 9 | 3 | 90 | 100 | — | 90 |
| 42 | 3 | — | 100 | — | 90 |
| 43 | 3 | 90 | 100 | — | 90 |
| 60 | 3 | 90 | 100 | 100 | 100 |
| 61 | 3 | 90 | 100 | 100 | 90 |
| 63 | 3 | 90 | 100 | 100 | 100 |
| 64 | 3 | 80 | 100 | 100 | 90 |
| 77 | 3 | 100 | 90 | 90 | 100 |
| 32 | 3 | 100 | 90 | 90 | 100 |
| 66 | 3 | 100 | 100 | 90 | 100 |
| 78 | 3 | 100 | 100 | 90 | 95 |
| 79 | 3 | 100 | 80 | — | 100 |

Beispiel A

Man vermischt 90 Gew.-Teile der Verbindung 1 mit 10 Gew.-Teilen N-Methyl-α-pyrrolidon und erhält eine Lösung, die zur Anwendung in Form kleinster Tropfen geeignet ist.

Beispiel B

20 Gew.-Teile der Verbindung 2 werden in einer Mischung gelöst, die aus 80 Gew.-Teilen Xylol, 10 Gew.-Teilen des Anlagerungsproduktes von 8 bis 10 Mol Äthylenoxid an 1 Mol Ölsäure-N-monoäthanolamid, 5 Gew.-Teilen Calciumsalz der Dodecylbenzolsulfonsäure und 5 Gew.-Teilen des Anlagerungsproduktes von 40 Mol Äthylenoxid an 1 Mol Ricinusöl besteht. Durch Ausgiessen und feines Verteilen der Lösung in 100000 Gew.-Teilen Wasser erhält man eine wässrige Dispersion, die 0,02 Gew.-% des Wirkstoffs enthält.

Beispiel C

20 Gew.-Teile der Verbindung 3 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Äthylenoxid an 1 Mol Isooctylphenol und 10 Gew.-Teilen des Anlagerungsproduktes von 0 Mol Äthylenoxid an 1 Mol Ricinusöl besteht. Durch Eingiessen und feines Verteilen der Lösung in 100000 Gew.-Teilen Wasser erhält man eine wässrige Dispersion, die 0,02 Gew.-% des Wirkstoffs enthält.

Beispiel D

20 Gew.-Teile der Verbindung 1 werden in einer Mischung gelöst, die aus 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 Mol Äthylenoxid an 1 Mol Ricinusöl besteht. Durch Eingiessen und feines Verteilen der Lösung in 100000 Gew.-Teilen Wasser erhält man eine wässrige Dispersion, die 0,02 Gew.-% des Wirkstoffs enthält.

Beispiel E

20 Gew.-Teile des Wirkstoffs 2 werden mit 3 Gew.-Teilen des Natriumsalzes der Diisobutylnaphthalin-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Beispiel F

3 Gew.-Teile der Verbindung 3 werden mit 97 Gew.-Teilen feinteiligem Kaolin innig vermischt. Man erhält auf diese Weise ein Stäubemittel, das 3 Gew.-% des Wirkstoffs enthält.

Beispiel G

30 Gew.-Teile der Verbindung 4 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel H

40 Gew.-Teile des Wirkstoffs 1 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wässrige Dispersion. Durch Verdünnen mit 100000 Gew.-Teilen Wasser erhält man eine wässrige Dispersion, die 0,04 Gew.-% Wirkstoff enthält.

Beispiel K

20 Teile des Wirkstoffs 2 werden mit 2 Teilen Calciumsalz der Dodecylbenzolsulfonsäure, 8 Teilen Fettalkohol-polyglykoläther, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

**Patentansprüche für die Vertragsstaaten: BE, CH, LI, DE, FR, GB, IT, NL, SE**

1. m-Anilidurethan der allgemeinen Formel

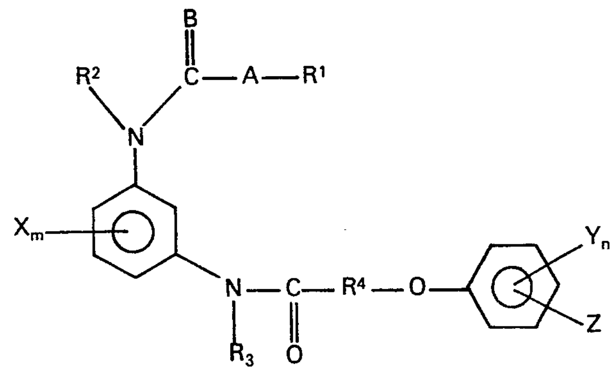

in der

A und B unabhängig voneinander Sauerstoff oder Schwefel

$R^1$ ggf. durch Halogen oder Alkoxy oder Alkoxycarbonyl oder Cyan substituiertes Alkyl oder ggf. durch Halogen substituiertes Alkenyl oder ggf. durch Halogen oder Alkoxy substituiertes Alkinyl oder ggf. durch Alkyl substituiertes Cycloalkyl oder Aryl

$R^2$ und $R^3$ jeweils unabhängig voneinander Wasserstoff, Alkyl, Alkoxyalkyl, Halogenalkyl

$R_4$ einen ggf. durch Alkyl, Alkoxyalkyl oder Halogenalkyl substituierten Alkylenrest mit 1 bis 6 Kohlenstoffatomen

X Wasserstoff, Alkyl, Halogenalkyl, Alkoxy, Halogen, Nitro oder Amino

Y Wasserstoff, Alkyl, Halogenalkyl, Alkoxyalkyl, Cycloalkyl, Aralkyl, Halogen, Alkoxy, Halogenalkoxy, Alkylthio, Nitro, Aryl, Thiocyanato,

Cyano, 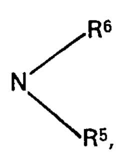 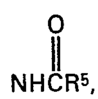 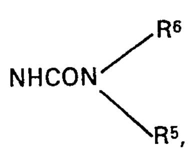 $COOR^5$, 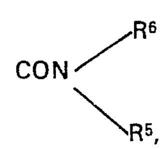

$SO_2R^5$, $OSO_2R^5$, $COR^5$, 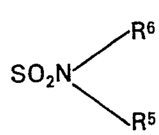 bedeutet,

wobei $R^5$ und $R^6$ jeweils unabhängig voneinander Wasserstoff bedeuten oder die für $R^1$ genannte Bedeutung haben,

Z den Rest 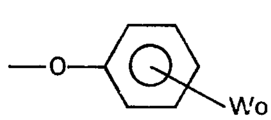

oder 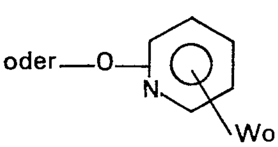 bedeutet,

wobei W unabhängig von Y die für Y genannten Bedeutungen hat und m, n und o die Zahlen 1 bis 4 bedeuten.

2. Herbizid, enthaltend ein m-Anilidurethan der allgemeinen Formel

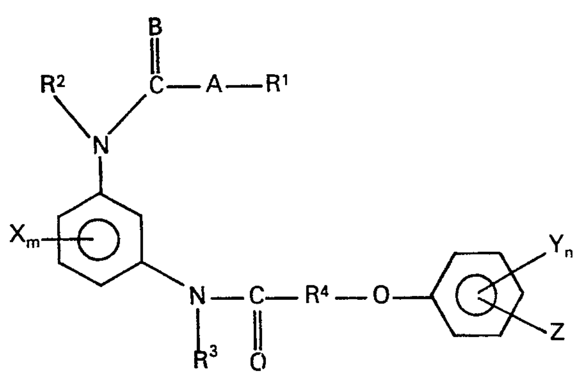

in der

A und B unabhängig voneinander Sauerstoff oder Schwefel
$R^1$ ggf. durch Halogen oder Alkoxy oder Alkoxycarbonyl oder Cyan substituiertes Alkyl oder ggf. durch Halogen substituiertes Alkenyl oder ggf. durch Halogen oder Alkoxy substituiertes Alkinyl oder ggf. durch Alkyl substituiertes Cycloalkyl oder Aryl
$R^2$ und $R^3$ jeweils unabhängig voneinander Wasserstoff, Alkyl, Alkoxyalkyl, Halogenalkyl
$R^4$ einen ggf. durch Alkyl, Alkoxyalkyl oder Halogenalkyl substituierten Alkylenrest mit 1 bis 6 Kohlenstoffatomen
X Wasserstoff, Alkyl, Halogenalkyl, Alkoxy, Halogen, Nitro oder Amino
Y Wasserstoff, Alkyl, Halogenalkyl, Alkoxyalkyl, Cycloalkyl, Aralkyl, Halogen, Alkoxy, Halogenalkoxy, Alkylthio, Nitro, Aryl, Thiocyanato,

Cyano, 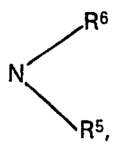 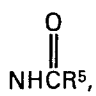 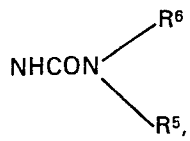 COOR$^5$, 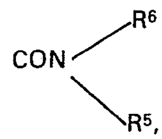

$SO_2R^5$, $OSO_2R^5$, COR$^5$, 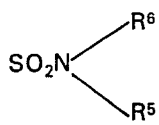 bedeutet,

wobei $R^5$ und $R^6$ jeweils unabhängig voneinander Wasserstoff bedeuten oder die für $R^1$ genannte Bedeutung haben,

Z den Rest 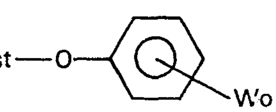 oder 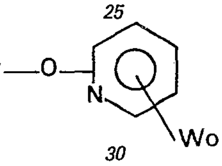 bedeutet,

wobei W unabhängig von Y die für Y genannten Bedeutungen hat und m, n und o die Zahlen 1 bis 4 bedeuten.

3. Herbizid, enthaltend einen festen oder flüssigen Trägerstoff und ein m-Anilidurethan der allgemeinen Formel

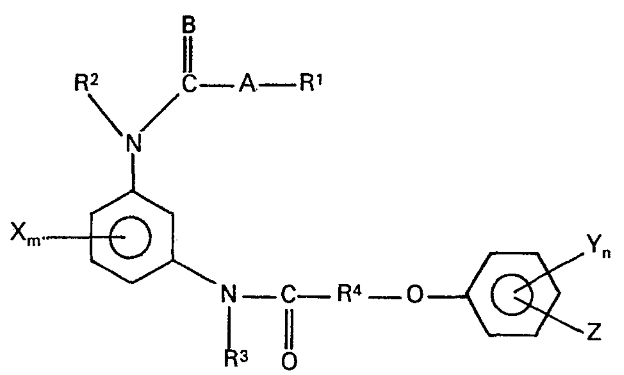

in der
A und B unabhängig voneinander Sauerstoff oder Schwefel
$R^1$ ggf. durch Halogen oder Alkoxy oder Alkoxycarbonyl oder Cyan substituiertes Alkyl oder ggf. durch Halogen substituiertes Alkenyl oder ggf. durch Halogen oder Alkoxy substituiertes Alkinyl oder ggf. durch Alkyl substituiertes Cycloalkyl oder Aryl
$R^2$ und $R^3$ jeweils unabhängig voneinander Wasserstoff, Alkyl, Alkoxyalkyl, Halogenalkyl
$R^4$ einen ggf. durch Alkyl, Alkoxyalkyl oder Halogenalkyl substituierten Alkylenrest mit 1 bis 6 Kohlenstoffatomen
X Wasserstoff, Alkyl, Halogenalkyl, Alkoxy, Halogen, Nitro oder Amino
Y Wasserstoff, Alkyl, Halogenalkyl, Alkoxyalkyl, Cycloalkyl, Aralkyl, Halogen, Alkoxy, Halogenalkoxy, Alkylthio, Nitro, Aryl, Thiocyanato,

Cyano, 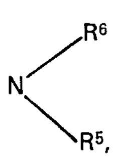 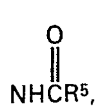 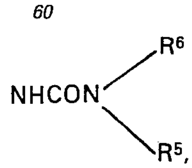 COOR$^5$, 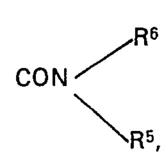

$SO_2R^5$, $OSO_2R^5$, $COR^5$, 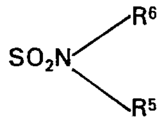 bedeutet,

wobei $R^5$ und $R^6$ jeweils unabhängig voneinander Wasserstoff bedeuten oder die für $R^1$ genannte Bedeutung haben,

Z den Rest 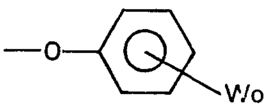

oder

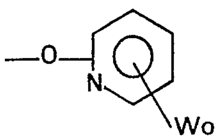 bedeutet,

wobei W unabhängig von Y die für Y genannten Bedeutungen hat und m, n oder o die Zahlen 1 bis 4 bedeuten.

4. Verfahren zur Bekämpfung unerwünschten Pflanzenwuchses, dadurch gekennzeichnet, dass man den Boden oder die Pflanzen behandelt mit einem m-Anilidurethan der allgemeinen Formel

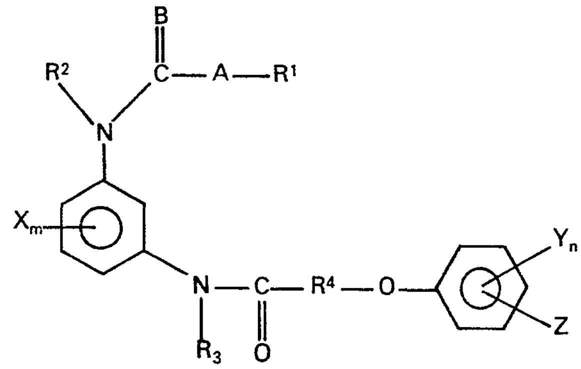

in der

A und B unabhängig voneinander Sauerstoff oder Schwefel

$R^1$ ggf. durch Halogen oder Alkoxy oder Alkoxycarbonyl oder Cyan substituiertes Alkyl oder ggf. durch Halogen substituiertes Alkenyl oder ggf. durch Halogen oder Alkoxy substituiertes Alkinyl oder ggf. durch Alkyl substituiertes Cycloalkyl oder Aryl

$R^2$ und $R^3$ jeweils unabhängig voneinander Wasserstoff, Alkyl, Alkoxyalkyl, Halogenalkyl

$R^4$ ggf. durch Alkyl, Alkoxyalkyl oder Halogenalkyl substituierten Alkylenrest mit 1 bis 6 Kohlenstoffatomen

X Wasserstoff, Alkyl, Halogenalkyl, Alkoxy, Halogen, Nitro oder Amino

Y Wasserstoff, Alkyl, Halogenalkyl, Alkoxyalkyl, Cycloalkyl, Aralkyl, Halogen, Alkoxy, Halogenalkoxy, Alkylthio, Nitro, Aryl, Thiocyanato,

Cyano, 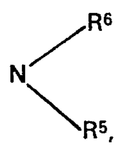 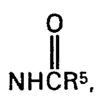 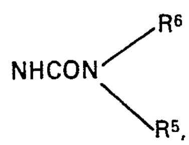 $COOR^5$, 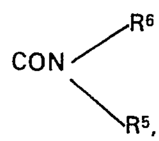

$SO_2R^5$, $OSO_2R^5$, $COR^5$, 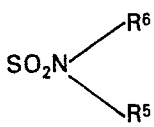 bedeutet,

wobei $R^5$ und $R^6$ jeweils unabhängig voneinander Wasserstoff bedeuten oder die für $R^1$ genannte Bedeutung haben,

Z den Rest 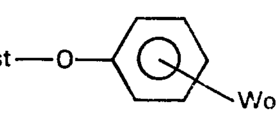

oder 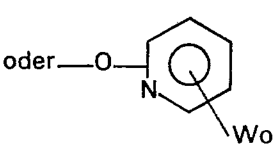 bedeutet,

wobei W unabhängig von Y die für Y genannten Bedeutungen hat und m, n oder o die Zahlen 1 bis 4 bedeuten.

5. m-Anilidurethan gemäss Anspruch 1, ausgewählt aus der Gruppe, bestehend aus den Verbindungen der Formeln

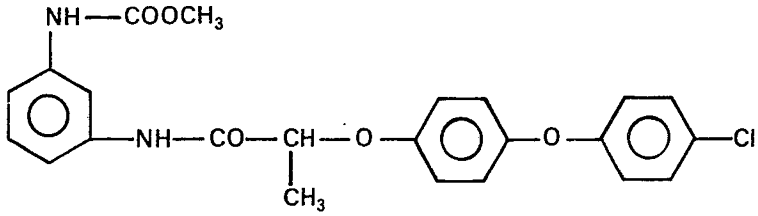

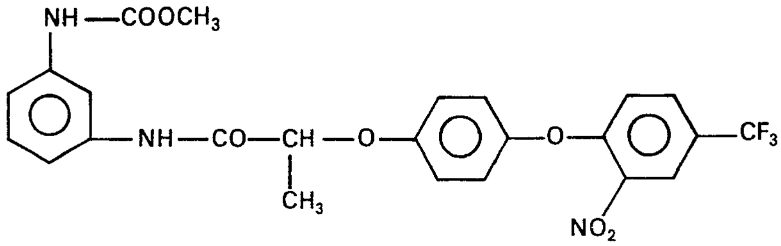

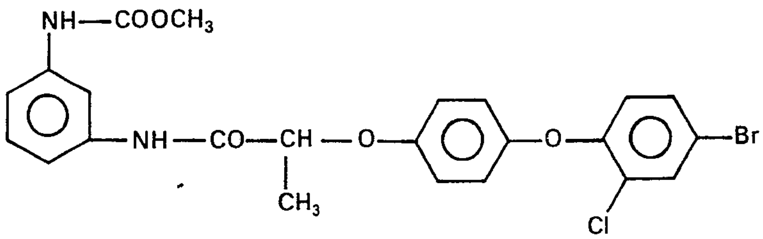

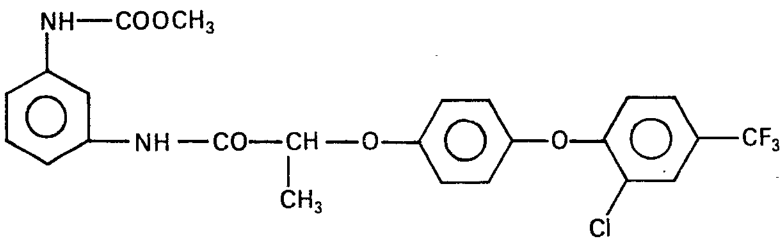

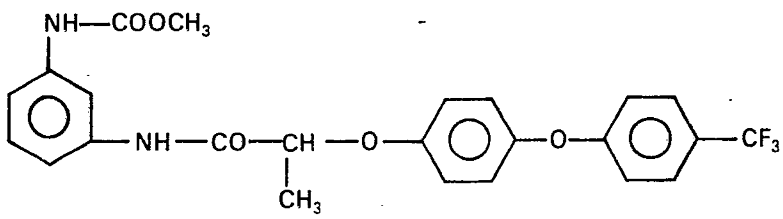

und

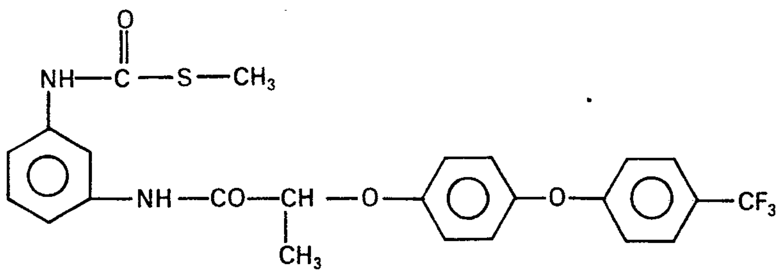

6. Herbizid gemäss Anspruch 2, enthaltend ein m-Anilidurethan, ausgewählt aus der Gruppe, bestehend aus den Verbindungen der Formeln

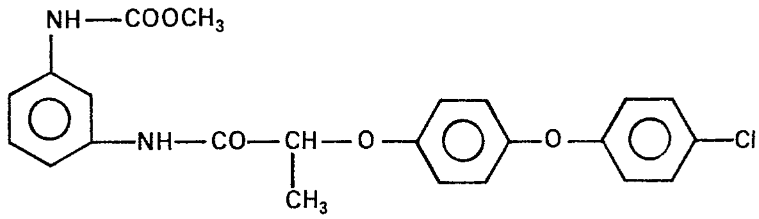

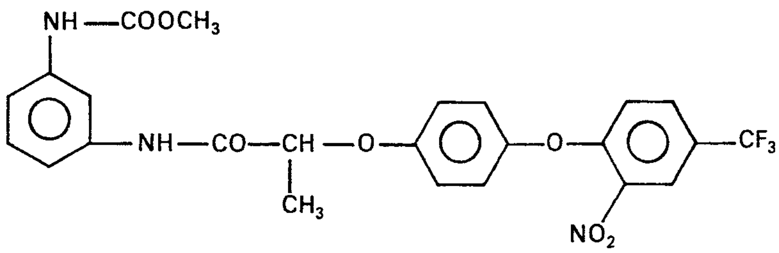

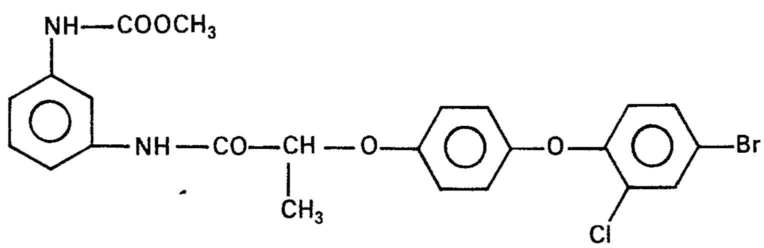

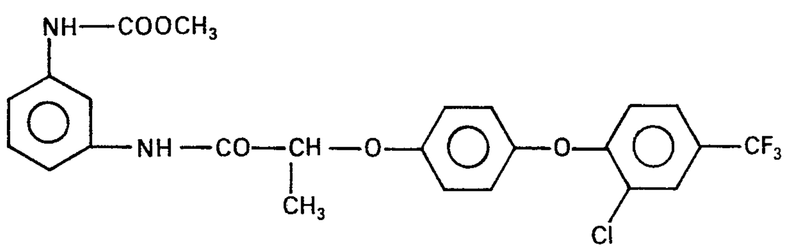

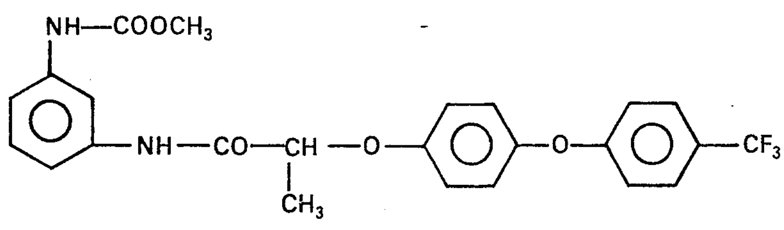

und

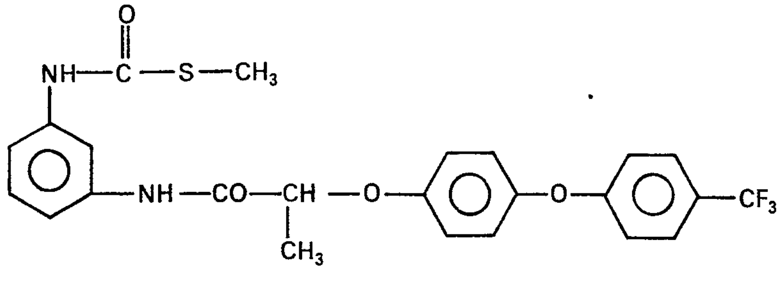

**Patentansprüche für den Vertragsstaat AT**

1. Herbizid, enthaltend als eine Komponente ein m-Anilidurethan der allgemeinen Formel

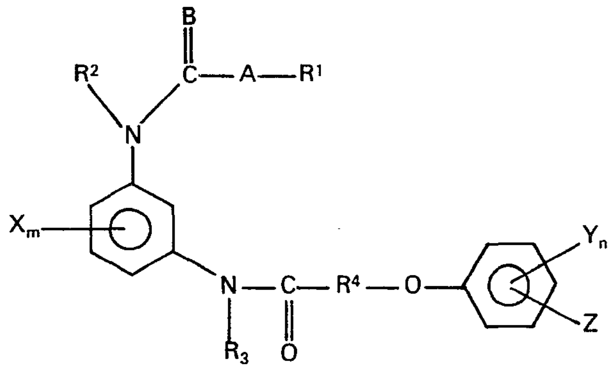

in der
A und B unabhängig voneinander Sauerstoff oder Schwefel
$R^1$ ggf. durch Halogen oder Alkoxy oder Alkoxycarbonyl oder Cyan substituiertes Alkyl oder ggf. durch Halogen substituiertes Alkenyl oder ggf. durch Halogen oder Alkoxy substituiertes Alkinyl oder ggf. durch Alkyl substituiertes Cycloalkyl oder Aryl
$R^2$ und $R^3$ jeweils unabhängig voneinander Wasserstoff, Alkyl, Alkoxyalkyl, Halogenalkyl,
$R^4$ einen ggf. durch Alkyl, Alkoxyalkyl oder Halogenalkyl substituierten Alkylenrest mit 1 bis 6 Kohlenstoffatomen
X Wasserstoff, Alkyl, Halogenalkyl, Alkoxy, Halogen, Nitro oder Amino
Y Wasserstoff, Alkyl, Halogenalkyl, Alkoxyalkyl, Cycloalkyl, Aralkyl, Halogen, Alkoxy, Halogenalkoxy, Alkylthio, Nitro, Aryl, Thiocyanato,

Cyano, 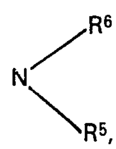 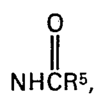 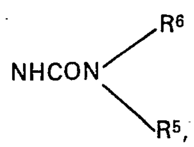 $COOR^5$, 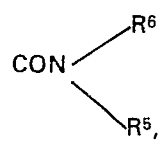

$SO_2R^5$, $OSO_2R^5$, $COR^5$, 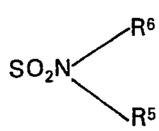 bedeutet,

wobei $R^5$ und $R^6$ jeweils unabhängig voneinander Wasserstoff bedeuten oder die für $R^1$ genannte Bedeutung haben,

Z den Rest 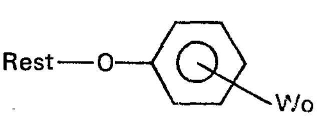 oder 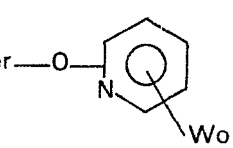 bedeutet,

wobei W unabhängig von Y die für Y genannten Bedeutungen hat und m, n oder o die Zahlen 1 bis 4 bedeuten.

2. Herbizid, enthaltend einen festen oder flüssigen Trägerstoff und ein m-Anilidurethanderivat der Formel

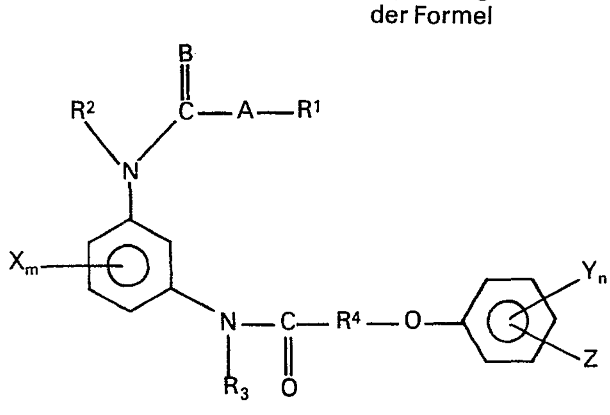

in der
A und B unabhängig voneinander Sauerstoff oder Schwefel
$R^1$ ggf. durch Halogen oder Alkoxy oder Alkoxycarbonyl oder Cyan substituiertes Alkyl oder ggf. durch Halogen substituiertes Alkenyl oder ggf. durch Halogen oder Alkoxy substituiertes Alkinyl oder ggf. durch Alkyl substituiertes Cycloalkyl oder Aryl
$R^2$ und $R^3$ jeweils unabhängig voneinander Wasserstoff, Alkyl, Alkoxyalkyl, Halogenalkyl
$R^4$ einen ggf. durch Alkyl, Alkoxyalkyl oder Halogenalkyl substituierten Alkylenrest mit 1 bis 6 Kohlenstoffatomen
X Wasserstoff, Alkyl, Halogenalkyl, Alkoxy, Halogen, Nitro oder Amino
Y Wasserstoff, Alkyl, Halogenalkyl, Alkoxyalkyl, Cycloalkyl, Aralkyl, Halogen, Alkoxy, Halogenalkoxy, Alkylthio, Nitro, Aryl, Thiocyanato,

Cyano, 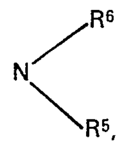 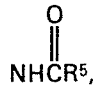 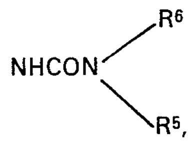 $COOR^5$, 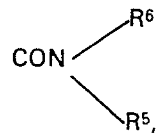

$SO_2R^5$, $OSO_2R^5$, $COR^5$, 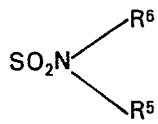 bedeutet,

wobei $R^5$ und $R^6$ jeweils unabhängig voneinander Wasserstoff bedeuten oder die für $R^1$ genannte Bedeutung haben,

Z den Rest 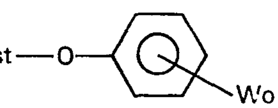 oder 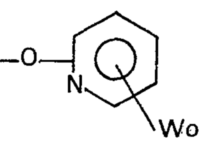 bedeutet,

wobei W unabhängig von Y die für Y genannten Bedeutungen hat und m, n und o die Zahlen 1 bis 4 bedeuten.

3. Verfahren zur Bekämpfung unerwünschten Pflanzenwuchses, dadurch gekennzeichnet, dass man den Boden oder die Pflanzen behandelt mit einem m-Anilidurethan der allgemeinen Formel

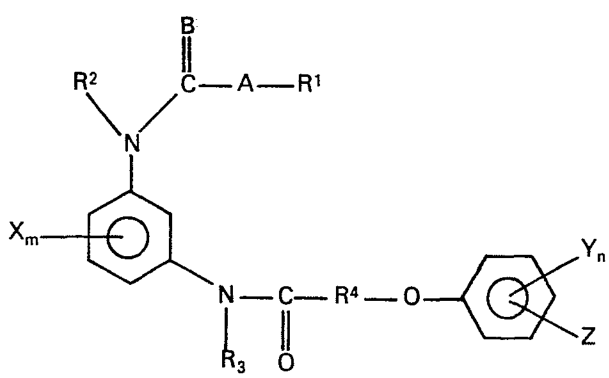

in der
A und B unabhängig voneinander Sauerstoff oder Schwefel
$R^1$ ggf. durch Halogen oder Alkoxy oder Alkoxycarbonyl oder Cyan substituiertes Alkyl oder ggf. durch Halogen substituiertes Alkenyl oder ggf. durch Halogen oder Alkoxy substituiertes Alkinyl oder ggf. durch Alkyl substituiertes Cycloalkyl oder Aryl
$R^2$ und $R^3$ jeweils unabhängig voneinander Wasserstoff, Alkyl, Alkoxyalkyl, Halogenalkyl
$R^4$ einen ggf. durch Alkyl, Alkoxyalkyl oder Halogenalkyl substituierten Alkylenrest mit 1 bis 6 Kohlenstoffatomen
X Wasserstoff, Alkyl, Halogenalkyl, Alkoxy, Halogen, Nitro oder Amino
Y Wasserstoff, Alkyl, Halogenalkyl, Alkoxyalkyl, Cycloalkyl, Aralkyl, Halogen, Alkoxy, Halogenalkoxy, Alkylthio, Nitro, Aryl, Thiocyanato,

Cyano, 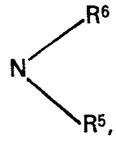 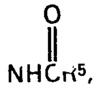 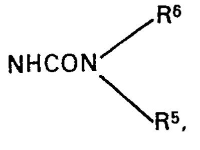 $COOR^5$, 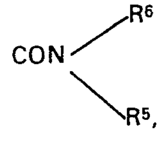

$SO_2R^5$, $OSO_2R^5$, $COR^5$, 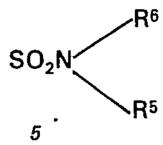 bedeutet,

wobei $R^5$ und $R^6$ jeweils unabhängig voneinander Wasserstoff bedeuten oder die für $R^1$ genannte Bedeutung haben,

Z den Rest 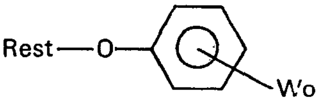 oder 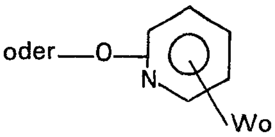 bedeutet,

wobei W unabhängig von Y die für Y genannten Bedeutungen hat und m, n oder o die Zahlen 1 bis 4 bedeuten.

4. Herbizid gemäss Anspruch 1, enthaltend ein m-Anilidurethan, ausgewählt aus der Gruppe, bestehend aus den Verbindungen der Formeln

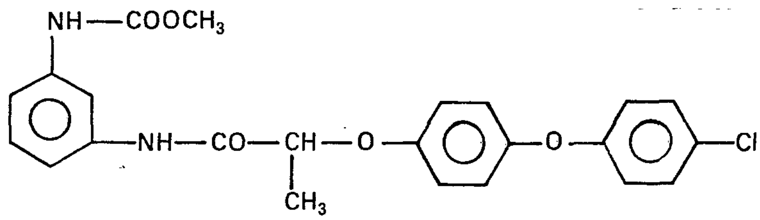

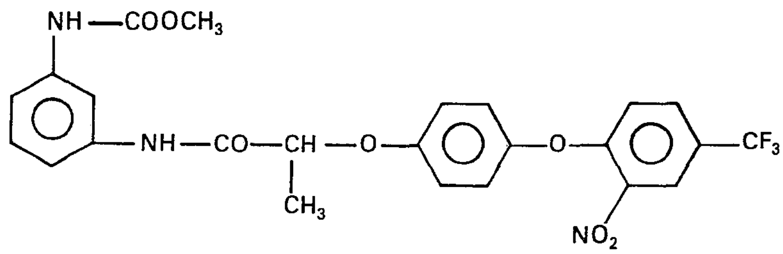

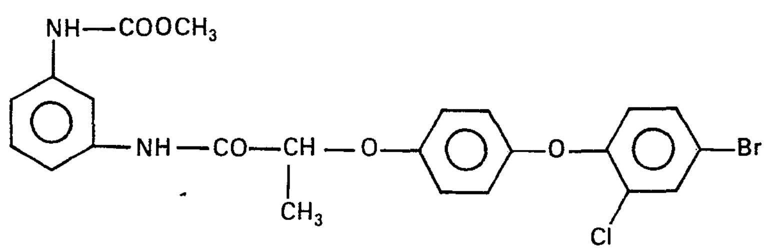

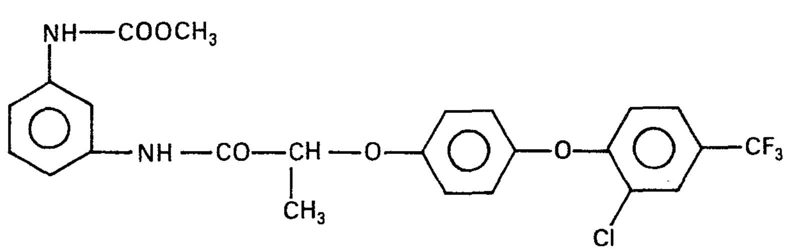

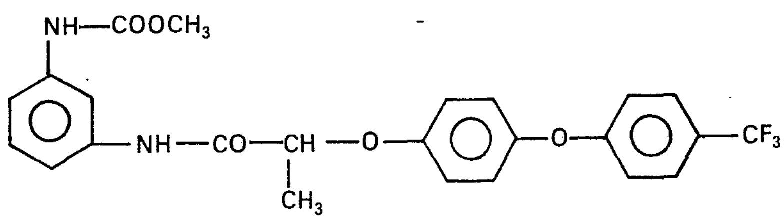

und

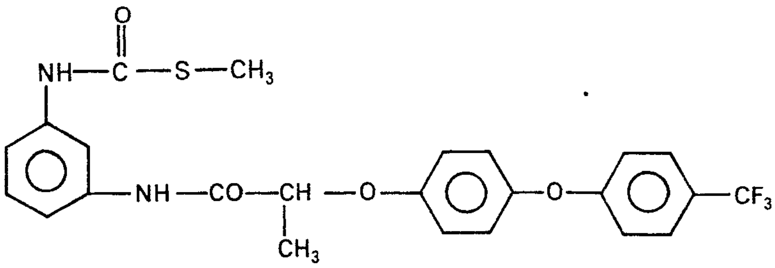

**Claims for the Contracting state: AT**

1. A herbicide containing, as one component, an m-anilido-urethane of the general formula

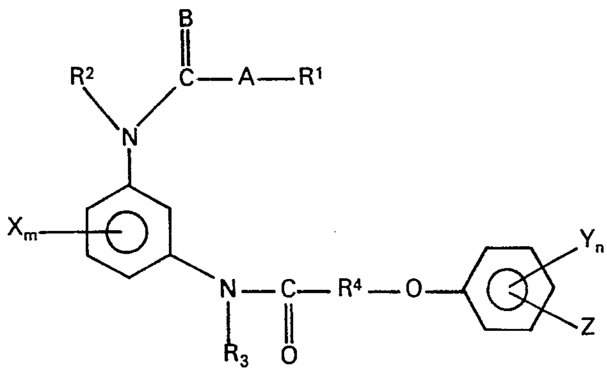

where

A and B independently of one another are oxygen or sulfur,

$R^1$ is alkyl, which is unsubstituted or substituted by halogen, alkoxy, alkoxycarbonyl or cyano, or is alkenyl which is unsubstituted or substituted by halogen, or is alkynyl which is unsubstituted or substituted by halogen or alkoxy, or is cycloalkyl which is unsubstituted or substituted by alkyl, or is aryl,

$R^2$ and $R^3$ are each independently of one another hydrogen, alkyl, alkoxyalkyl or haloalkyl,

$R^4$ is alkylene of 1 to 6 carbon atoms which is unsubstituted or substituted by alkyl, alkoxyalkyl or haloalkyl,

X is hydrogen, alkyl, haloalkyl, alkoxy, halogen, nitro or amino,

Y is hydrogen, alkyl, haloalkyl, alkoxyalkyl, cycloalkyl, aralkyl, halogen, alkoxy, haloalkoxy, alkylthio, nitro, aryl, thiocyanato, cyano

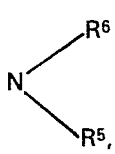 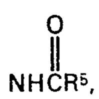 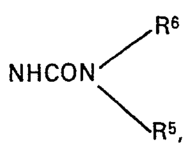 $COOR^5$, 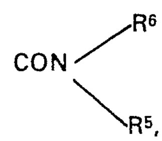

$SO_2R^5$, $OSO_2R^5$, $COR^5$ or 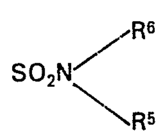 where

$R^5$ and $R^6$ are each, independently of one another, hydrogen or have the meanings given for $R^1$, and

Z is 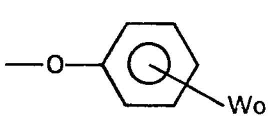 or 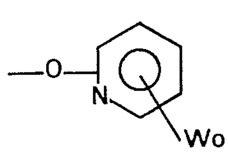

where W has, independently of Y, the meanings given for Y and m, n and o are numbers from 1 to 4.

2. A herbicide containing a solid or liquid carrier and an m-anilido-urethane of the formula

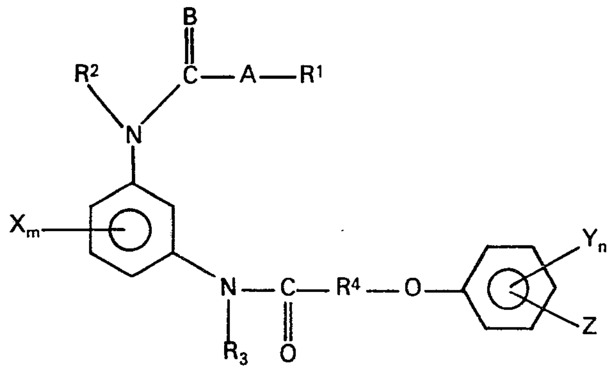

where
A and B independently of one another are oxygen or sulfur,
$R^1$ is alkyl, which is unsubstituted or substituted by halogen, alkoxy, alkoxycarbonyl or cyano, or is alkenyl which is unsubstituted or substituted by halogen, or is alkynyl which is unsubstituted or substituted by halogen or alkoxy, or is cycloalkyl which is unsubstituted or substituted by alkyl, or is aryl,
$R^2$ and $R^3$ are each independently of one another hydrogen, alkyl, alkoxyalkyl or haloalkyl,
$R^4$ is alkylene of 1 to 6 carbon atoms which is unsubstituted or substituted by alkyl, alkoxyalkyl or haloalkyl,
X is hydrogen, alkyl, haloalkyl, alkoxy, halogen, nitro or amino,
Y is hydrogen, alkyl, haloalkyl, alkoxyalkyl, cycloalkyl, aralkyl, halogen, alkoxy, haloalkoxy, alkylthio, nitro, aryl, thiocyanato, cyano

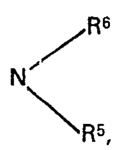 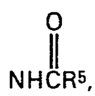 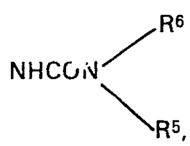 $COOR^5$, 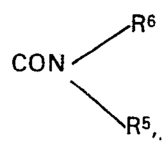

$SO_2R^5$, $OSO_2R^5$, $COR^5$ or 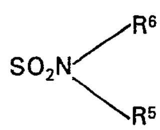

where
$R^5$ and $R^6$ are each, independently of one another, hydrogen or have the meanings given for $R^1$; and

Z is 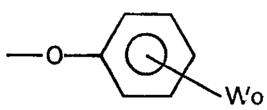 or 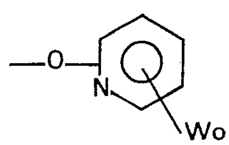

where W has, independently of Y, the meanings given for Y and m, n and o are numbers from 1 to 4.

3. A process for combating the growth of unwanted plants, characterized in that the soil or the plants are treated with an m-anilido-urethane of the general formula

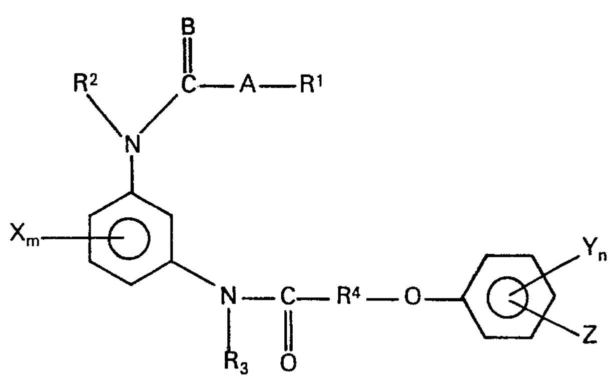

where
A and B independently of one another are oxygen or sulfur,
$R^1$ is alkyl, which is unsubstituted or substituted by halogen, alkoxy, alkoxycarbonyl or cyano, or is alkenyl which is unsubstituted or substituted by

halogen, or is alkynyl which is unsubstituted or substituted by halogen or alkoxy, or is cycloalkyl which is unsubstituted or substituted by alkyl, or is aryl,
$R^2$ and $R^3$ are each independently of one another hydrogen, alkyl, alkoxyalkyl or haloalkyl,
$R^4$ is alkylene of 1 to 6 carbon atoms which is unsubstituted or substituted by alkyl, alkoxyalkyl or haloalkyl,
X is hydrogen, alkyl, haloalkyl, alkoxy, halogen, nitro or amino,
Y is hydrogen, alkyl, haloalkyl, alkoxyalkyl, cycloalkyl, aralkyl, halogen, alkoxy, haloalkoxy, alkylthio, nitro, aryl, thiocyanato, cyano

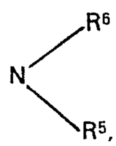 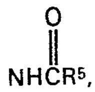, 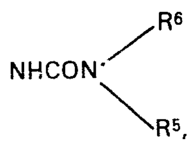 $COOR^5$, 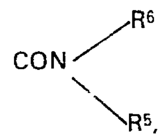

$SO_2R^5$, $OSO_2R^5$, $COR^5$ or 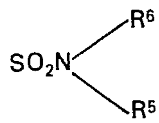

where
$R^5$ and $R^6$ are each, independently of one another, hydrogen or have the meanings given for $R^1$, and

Z is 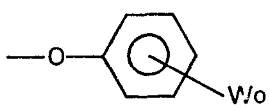 or 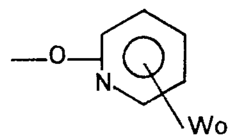

where W has, independently of Y, the meanings given for Y and m, n and o are numbers from 1 to 4.

4. A herbicide as claimed in claim 1, containing an m-anilido-urethano selected from the group consisting of the compounds of the formulae

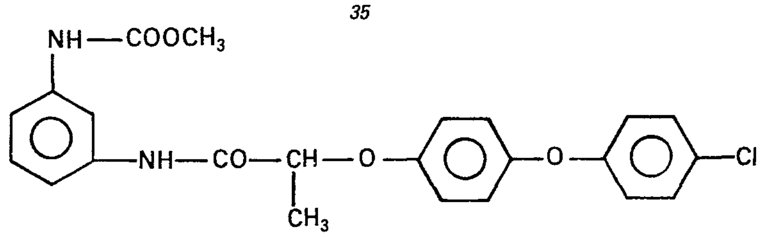

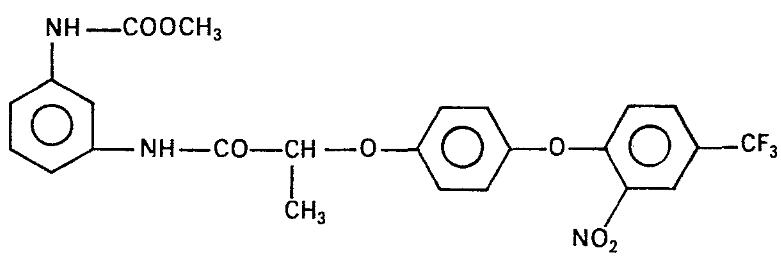

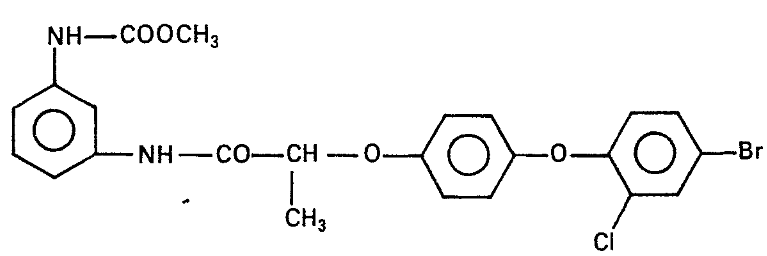

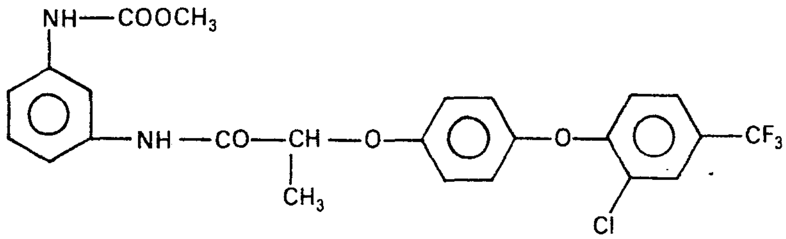

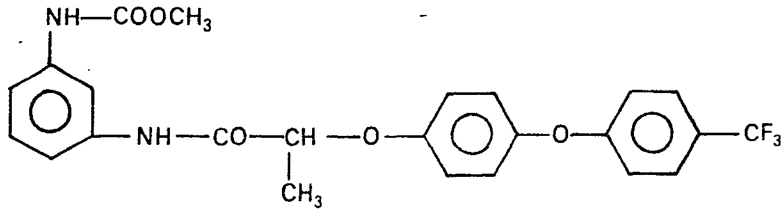

and

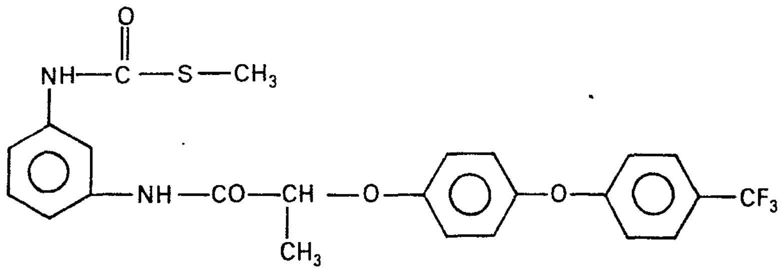

**Claims for the Contracting states: BE, CH, LI, DE, FR, GB, IT, NL, SE**

1. An m-anilido-urethane of the general formula

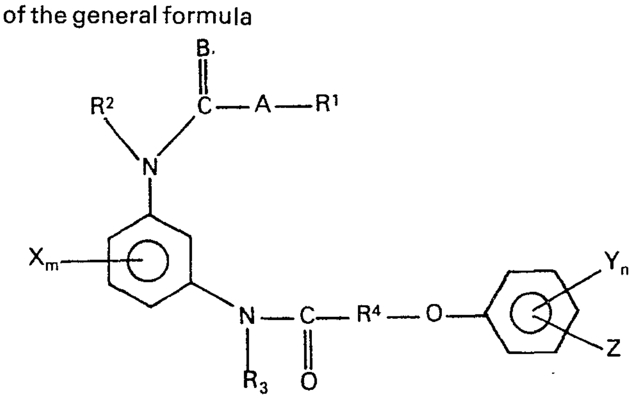

where
A and B independently of one another are oxygen or sulfur,
$R^1$ is alkyl, which is unsubstituted or substituted by halogen, alkoxy, alkoxycarbonyl or cyano, or is alkenyl which is unsubstituted or substituted by halogen, or is alkynyl which is unsubstituted or substituted by halogen or alkoxy, or is cycloalkyl which is unsubstituted or substituted by alkyl, or is aryl,
$R^2$ and $R^3$ are each independently of one another hydrogen, alkyl, alkoxyalkyl or haloalkyl
$R^4$ is alkylene of 1 to 6 carbon atoms which is unsubstituted or substituted by alkyl, alkoxyalkyl or haloalkyl,
X is hydrogen, alkyl, haloalkyl, alkoxy, halogen, nitro or amino,
Y is hydrogen, alkyl, haloalkyl, alkoxyalkyl, cycloalkyl, aralkyl, halogen, alkoxy, haloalkoxy, alkylthio, nitro, aryl, thiocyanato, cyano,

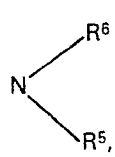
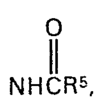
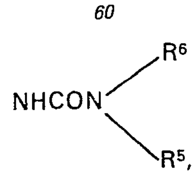
$COOR^5$,
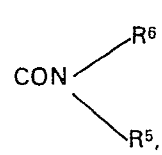

$SO_2R^5$, $OSO_2R^5$, $COR^5$, 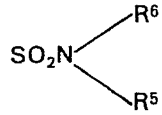

where
$R^5$ and $R^6$ are each, independently of one another, hydrogen or have the meanings given for $R^1$, and

Z is 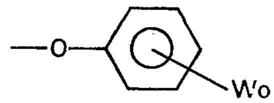 or 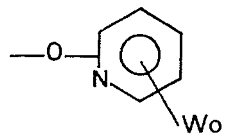

where W has, independently of Y, the meanings given for Y and m, n and o are numbers from 1 to 4.

2. A herbicide containing an m-anilido-urethane of the general formula

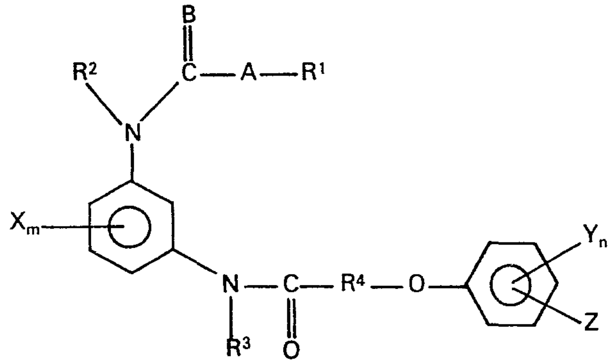

where
A and B independently of one another are oxygen or sulfur,
$R^1$ is alkyl, which is unsubstituted or substituted by halogen, alkoxy, alkoxycarbonyl or cyano, or is alkenyl which is unsubstituted or substituted by halogen, or is alkynyl which is unsubstituted or substituted by halogen or alkoxy, or is cycloalkyl which is unsubstituted or substituted by alkyl, or is aryl,
$R^2$ and $R^3$ are each independently of one another hydrogen, alkyl, alkoxyalkyl or haloalkyl,
$R^4$ is alkylene of 1 to 6 carbonatoms which is unsubstituted or substituted by alkyl, alkoxyalkyl or haloalkyl,
X is hydrogen, alkyl, haloalkyl, alkoxy, halogen, nitro or amino,
Y is hydrogen, alkyl, haloalkyl, alkoxyalkyl, cycloalkyl, aralkyl, halogen, alkoxy, haloalkoxy, alkylthio, nitro, aryl, thiocyanato, cyano,

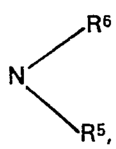 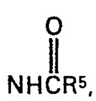 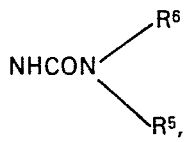 $COOR^5$, 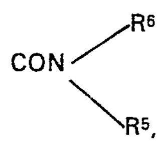

$SO_2R^5$, $OSO_2R^5$, $COR^5$ or 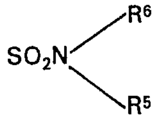

where
$R^5$ and $R^6$ are each, independently of one another, hydrogen or have the meanings given for $R^1$, and

Z is 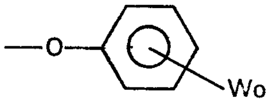 or 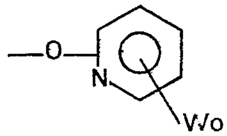

where W has, independently of Y, the meanings given for Y and m, n and o are numbers from 1 to 4.

3. A herbicide containing a solid or liquid carrier and an m-anilido-urethane of the general formula

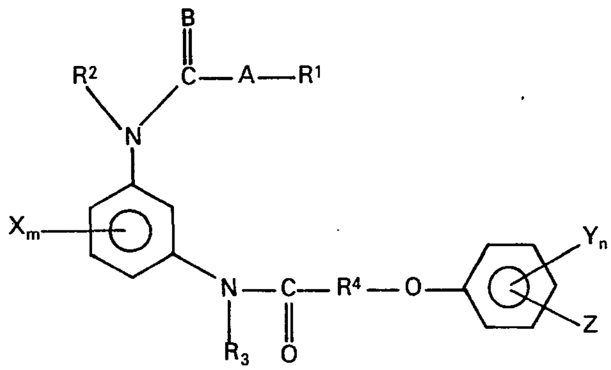

where

A and B independently of one another are oxygen or sulfur,

$R^1$ is alkyl, which is unsubstituted or substituted by halogen, alkoxy, alkoxycarbonyl or cyano, or is alkenyl which is unsubstituted or substituted by halogen, or is alkynyl which is unsubstituted or substituted by halogen or alkoxy, or is cycloalkyl which is unsubstituted or substituted by alkyl, or is aryl,

$R^2$ and $R^3$ are each independently of one another hydrogen, alkyl, alkoxyalkyl or haloalkyl,

$R^4$ is alkylene of 1 to 6 carbon atoms which is unsubstituted or substituted by alkyl, alkoxyalkyl or haloalkyl,

X is hydrogen, alkyl, haloalkyl, alkoxy, halogen, nitro or amino,

Y is hydrogen, alkyl, haloalkyl, alkoxyalkyl, cycloalkyl, aralkyl, halogen, alkoxy, haloalkoxy, alkylthio, nitro, aryl, thiocyanato, cyano

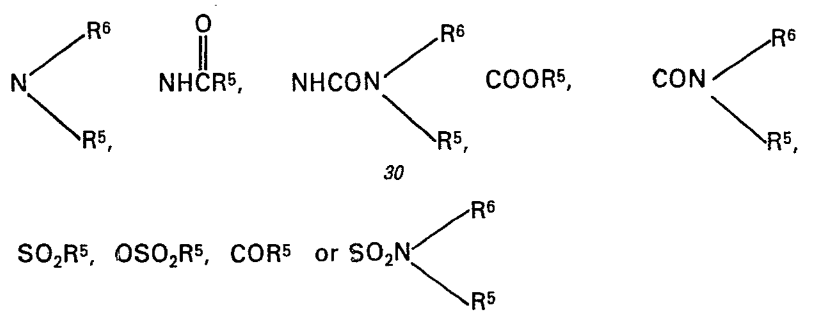

where $R^5$ and $R^6$ are each, independently of one another, hydrogen or have the meanings given for $R^1$, and

Z is 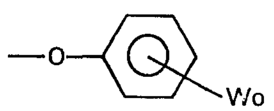 or 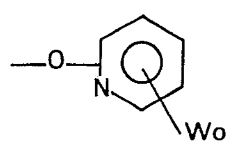

where W has, independently of Y, the meanings given for Y and m, n and o are numbers from 1 to 4.

4. A process for combating the growth of unwanted plants, characterized in that the soil or the plants are treated with an m-anilido-urethane of the general formula

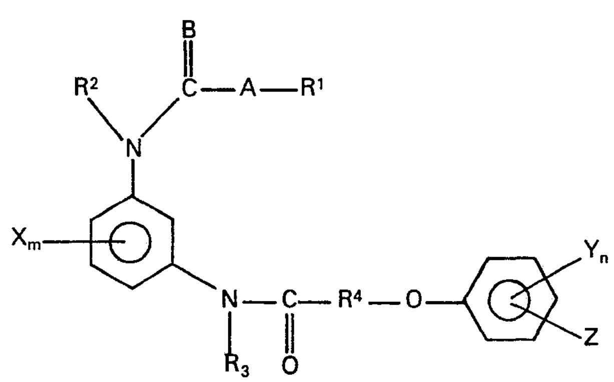

where

A and B independently of one another are oxygen or sulfur,

$R^1$ is alkyl, which is unsubstituted or substituted by halogen, alkoxy, alkoxycarbonyl or cyano, or is alkenyl which is unsubstituted or substituted by

halogen, or is alkynyl which is unsubstituted or substituted by halogen or alkoxy, or is cycloalkyl which is unsubstituted or substituted by alkyl, or is aryl,
$R^2$ and $R^3$ are each independently of one another hydrogen, alkyl, alkoxyalkyl or haloalkyl,
$R^4$ is alkylene of 1 to 6 carbon atoms which is unsubstituted or substituted by alkyl, alkoxyalkyl or haloalkyl,
X is hydrogen, alkyl, haloalkyl, alkoxy, halogen, nitro or amino,
Y is hydrogen, alkyl, haloalkyl, alkoxyalkyl, cycloalkyl, aralkyl, halogen, alkoxy, haloalkoxy, alkylthio, nitro, aryl, thiocyanato, cyano,

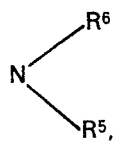 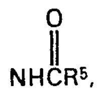 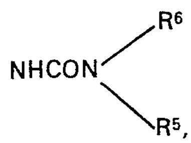 $COOR^5$, 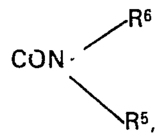

$SO_2R^5$, $OSO_2R^5$, $COR^5$ or 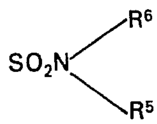

where $R^5$ and $R^6$ are each, independently of one another, hydrogen or have the meanings given for $R^1$, and

Z is 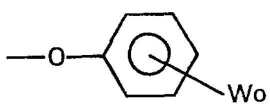 or 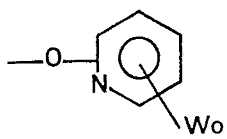

where W has, independently of Y, the meanings given for Y and m, n and o are numbers from 1 to 4.

5. An m-anilido-urethane as claimed in claim 1, selected from the group consisting of the compounds of the formulae

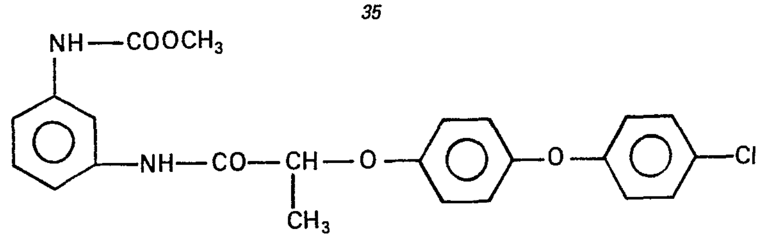

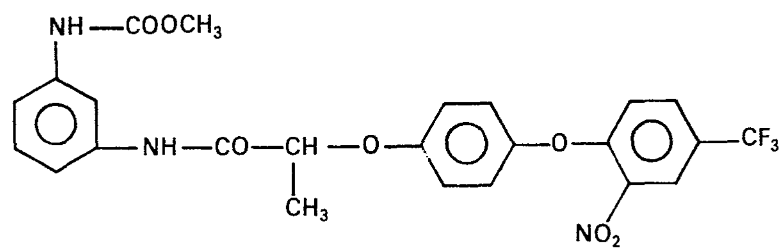

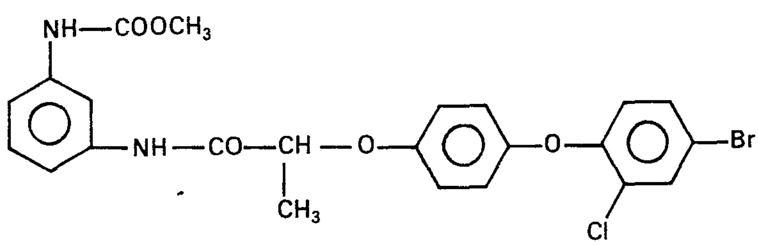

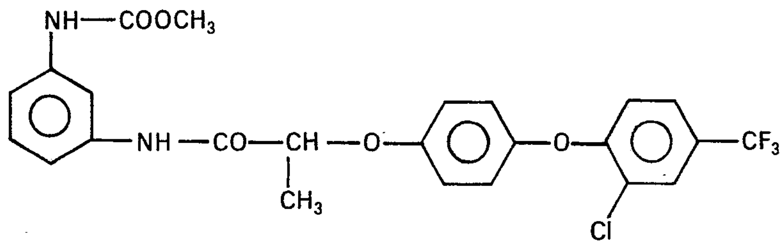

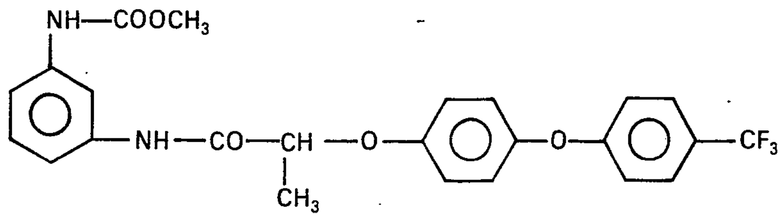

and

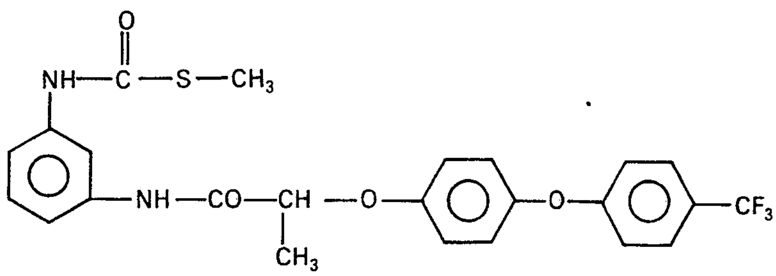

6. A herbicide as claimed in claim 2, containing an m-anilido-urethane selected from the group consisting of the compounds of the formulae

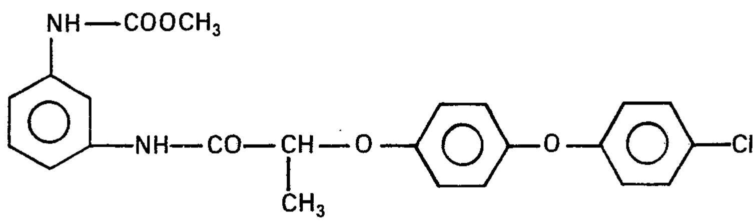

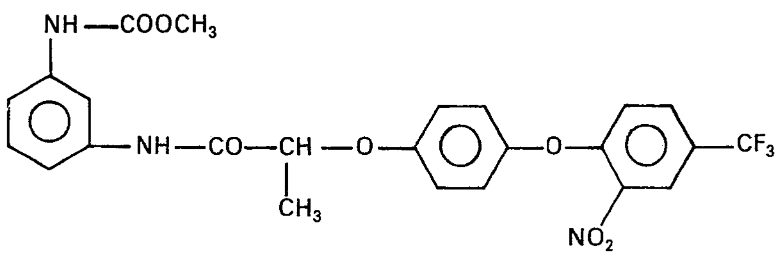

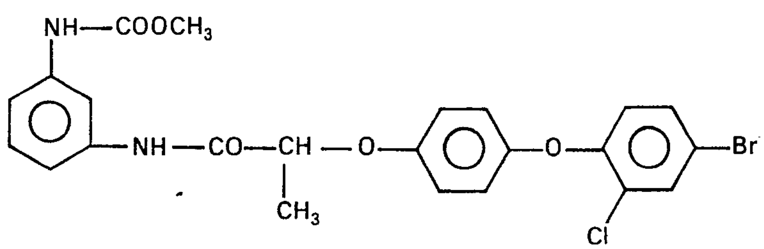

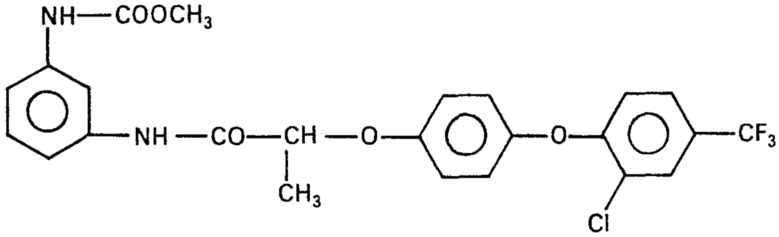

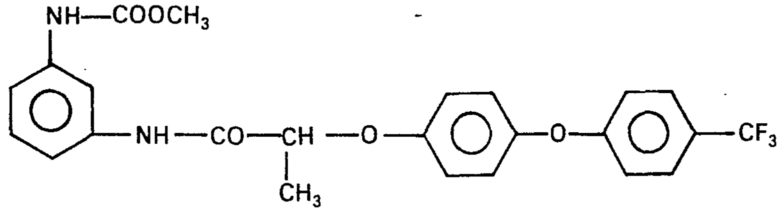

and

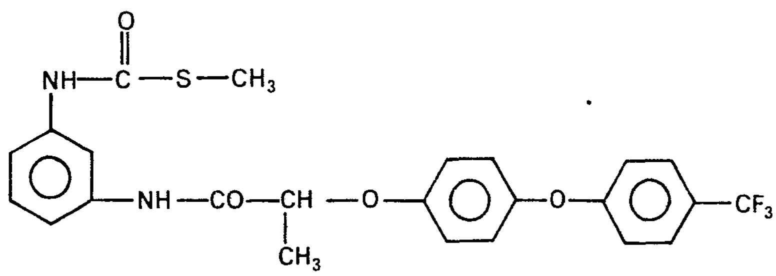

**Revendications pour les Etats contractants: BE, CH, LI, DE, FR, GB, IT, NL, SE**

1. m-aniliduréthane de formule générale:

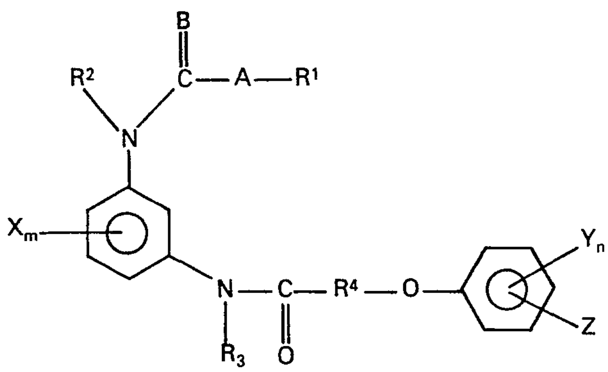

dans laquelle
A et B représentent, indépendamment l'un de l'autre, oxygène ou soufre,
$R^1$ alkyle, éventuellement substitué par halogène, alcoxy, alcoxycarbonyle ou cyano, ou alcényle, éventuellement substitué par halogène, ou alcinyle, éventuellement substitué par halogène ou alcoxy, ou cycloalkyle, éventuellement substitué par alkyle, ou aryle,
$R^2$ et $R^3$, chacun, indépendamment l'un de l'autre, hydrogène, alkyle, alcoxyalkyle, halogénalkyle,
$R^4$ un reste alkylène à 1 à 6 atomes de carbone, éventuellement substitué par alkyle, alcoxyalkyle ou halogénalkyle,
X hydrogène, alkyle, halogénalkyle, alcoxy, halogène, nitro ou amino,
Y hydrogène, alkyle, halogénalkyle, alcoxyalkyle, cycloalkyle, aralkyle, halogène, alcoxy, halogénalcoxy, alkylthio, nitro, aryle, thiocyanato, cyano,

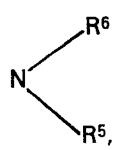 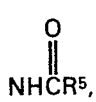 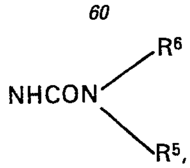 $COOR^5$, 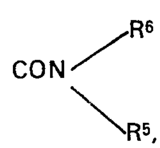

$SO_2R^5$, $OSO_2R^5$, $COR^5$, 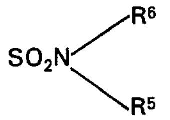

$R^5$ et $R^6$ représentant, indépendamment l'un de l'autre, hydrogène, ou ayant la signification donnée pour $R^1$,

Z le reste 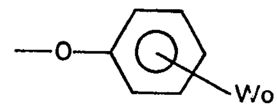 ou 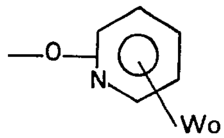

W ayant, indépendamment de Y, la signification donnée pour Y, et m, n et o représentent des nombres de 1 à 4.

2. Herbicide contenant un m-aniliduréthane de formule générale

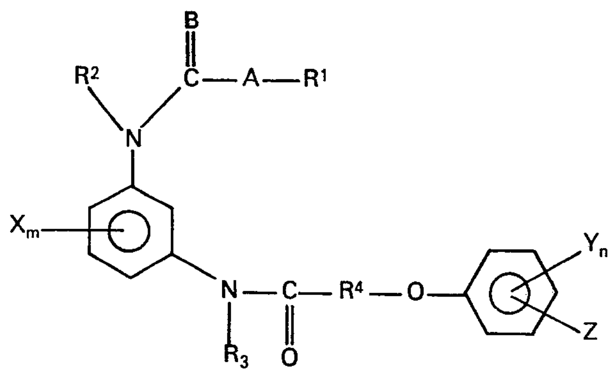

dans laquelle
A et B représentent, indépendamment l'un de l'autre, oxygène ou soufre,
$R^1$ alkyle, éventuellement substitué par halogène, alcoxy, alcoxycarbonyle ou cyano, ou alcényle, éventuellement substitué par halogène, ou alcinyle, éventuellement substitué par halogène ou alcoxy, ou cycloalkyle, éventuellement substitué par alkyle, aryle,
$R^2$ et $R^3$, chacun, indépendamment l'un de l'autre, hydrogène, alkyle, alcoxyalkyle, halogénalkyle,
$R^4$ un reste alkylène à 1 à 6 atomes de carbone, éventuellement substitué par alkyle, alcoxyalkyle ou halogénalkyle,
X hydrogène, alkyle, halogénalkyle, alcoxy, halogène, nitro ou amino,
Y hydrogène, alkyle, halogénalkyle, alcoxyalkyle, cycloalkyle, aralkyle, halogène, alcoxy, halogénalcoxy, alkylthio, nitro, aryle, thiocyanato, cyano,

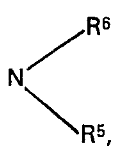 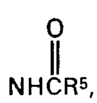 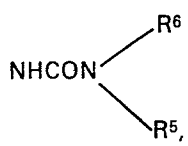 $COOR^5$, 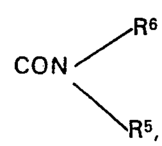

$SO_2R^5$, $OSO_2R^5$, $COR^5$, 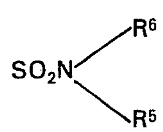

$R^5$ et $R^6$ représentant, indépendamment l'un de l'autre, hydrogène ou ayant la signification donnée pour $R^1$,

Z le reste 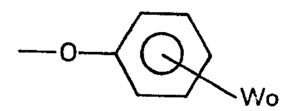 ou 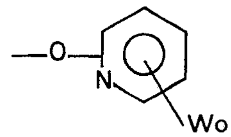

W ayant, indépendamment de Y, la signification donnée pour Y, et m, n et o représentant des nombres de 1 à 4.

3. Herbicide contenant un support solide ou liquide et un m-aniliduréthane de formule générale

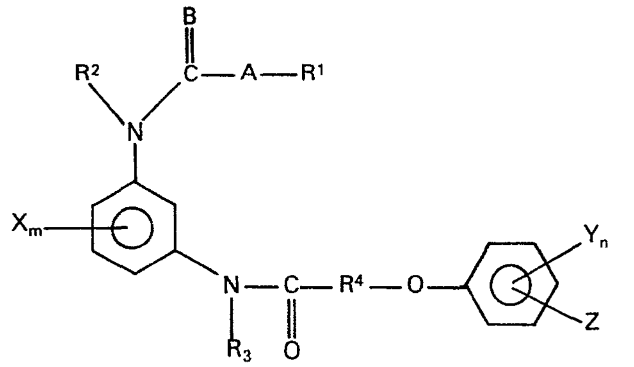

dans laquelle
A et B représentent, indépendamment l'un de l'autre, oxygène ou soufre,
$R^1$ alkyle, éventuellement substitué par halogène, alcoxy, alcoxycarbonyle ou cyano, ou alcényle, éventuellement substitué par halogène, ou alcinyle, éventuellement substitué par halogène ou alcoxy, ou cycloalkyle, éventuellement substitué par alkyle, ou aryle,
$R^2$ et $R^3$, chacun, indépendamment l'un de l'autre, hydrogène, alkyle, alcoxyalkyle, halogénalkyle,
$R^4$ un reste alkylène à 1 à 6 atomes de carbone, éventuellement substitué par alkyle, alcoxyalkyle ou halogénalkyle,
X hydrogène, alkyle, halogénalkyle, alcoxy, halogène, nitro ou amino,
Y hydrogène, alkyle, halogénalkyle, alcoxyalkyle, cycloalkyle, aralkyle, halogène, alcoxy, halogénalcoxy, alkylthio, nitro, aryle, thiocyanato, cyano,

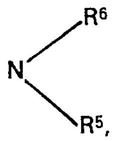 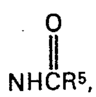 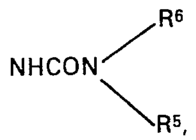 $COOR^5$, 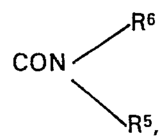

$SO_2R^5$, $OSO_2R^5$, $COR^5$, 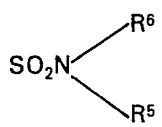

$R^5$ et $R^6$ représentant, indépendamment l'un de l'autre, hydrogène ou ayant la signification donnée pour $R^1$,

Z le reste 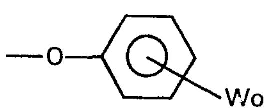 ou 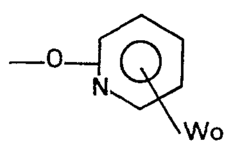

W ayant, indépendamment de Y, la signification donnée pour Y, et m, n et o représentant des nombres de 1 à 4.

4. Procédé de lutte contre la croissance indésirable des plantes, caractérisé par le fait qu'on traite le sol ou les plantes avec un m-aniliduréthane de formule générale

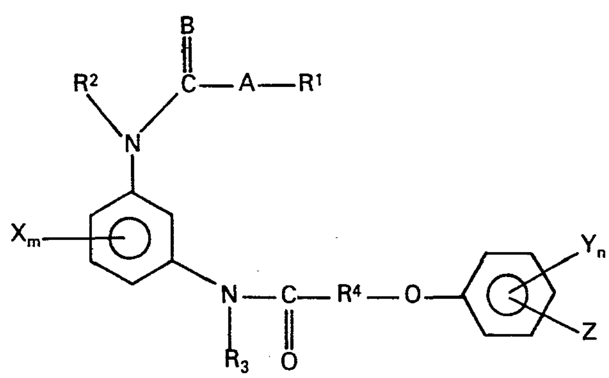

dans laquelle
A et B représentent, indépendamment l'un de l'autre, oxygène ou soufre,
$R^1$ alkyle, éventuellement substitué par halogène, alcoxy, alcoxycarbonyle ou cyano, ou alcényle, éventuellement substitué par halogène, ou alcinyle, éventuellement substitué par halogène, ou alcoxy, ou cycloalkyle, éventuellement substitué par alkyle, ou aryle,
$R^2$ et $R^3$, chacun, indépendamment l'un de l'autre, hydrogène, alkyle, alcoxyalkyle, halogénalkyle,
$R^4$ un reste alkylène à 1 à 6 atomes de carbone, éventuellement substitué par alkyle, alcoxyalkyle ou halogénalkyle,
X hydrogène, alkyle, halogénalkyle, alcoxy, halogène, nitro ou amino,
Y hydrogène, alkyle, halogénalkyle, alcoxyalkyle, cycloalkyle, aralkyle, halogène, alcoxy, halogénalcoxy, alkylthio, nitro, aryle, thiocyanato, cyano,

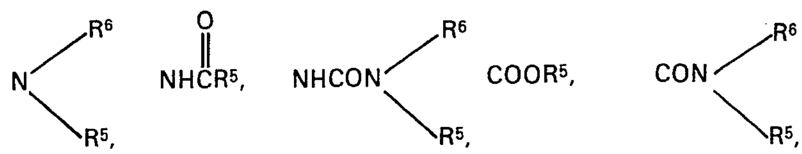

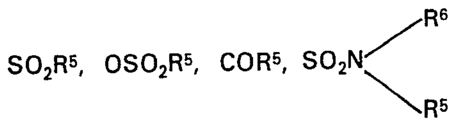

$R^5$ et $R^6$ représentant, indépendamment l'un de l'autre, hydrogène ou ayant la signification donnée pour $R^1$,

Z le reste 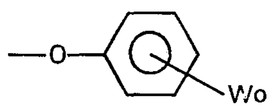 ou 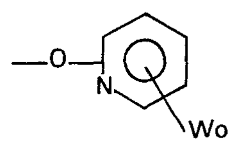

W ayant, indépendamment de Y, la signification donnée pour Y, et m, n et o représentant des nombres de 1 à 4.

5. m-aniliduréthane selon la revendication 1, choisi dans le groupe constitué par les composés de formule

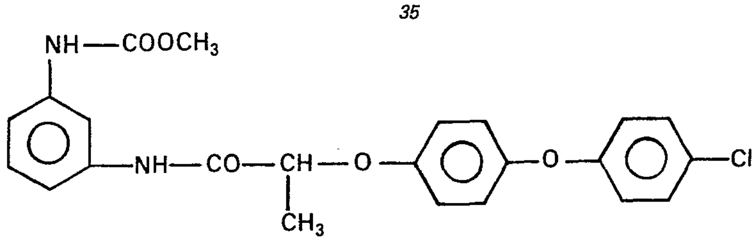

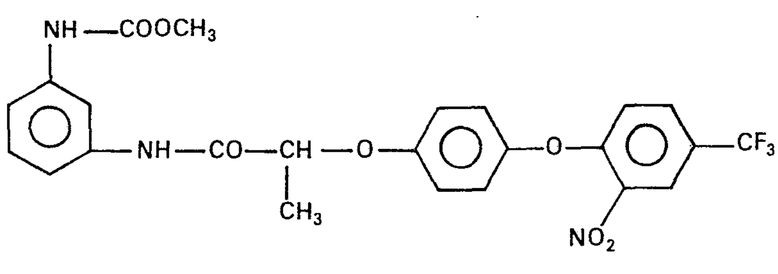

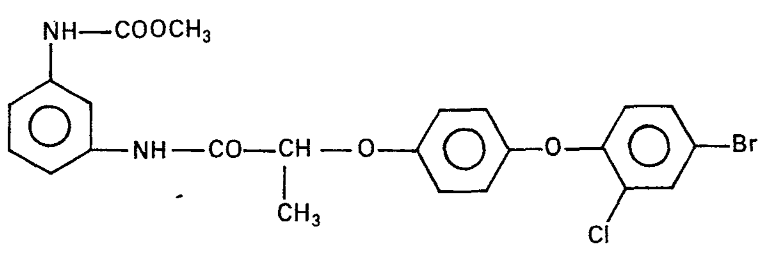

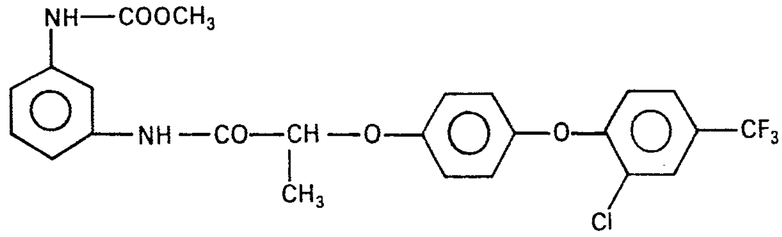

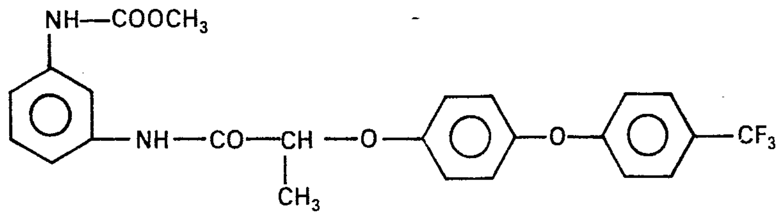

et

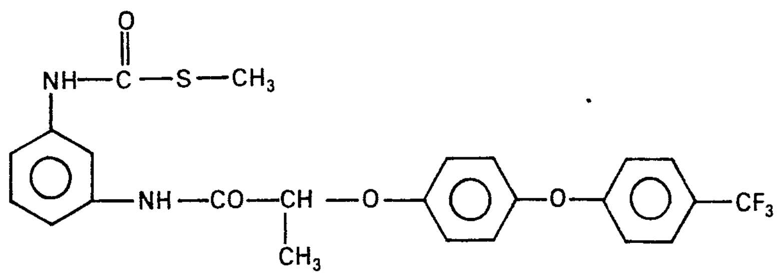

6. Herbicide selon la revendication 2 contenant un m-aniliduréthane choisi dans le groupe constitué par les composés de formule

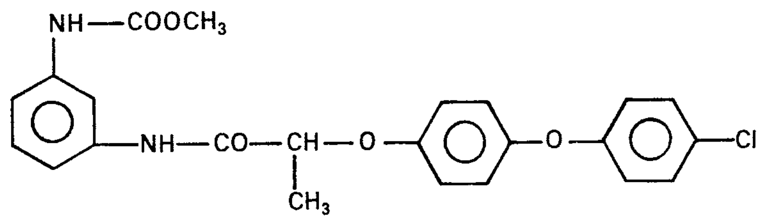

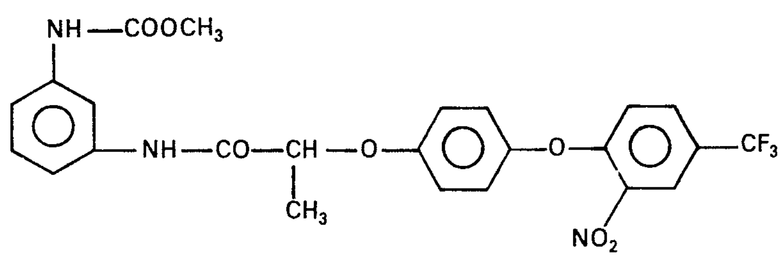

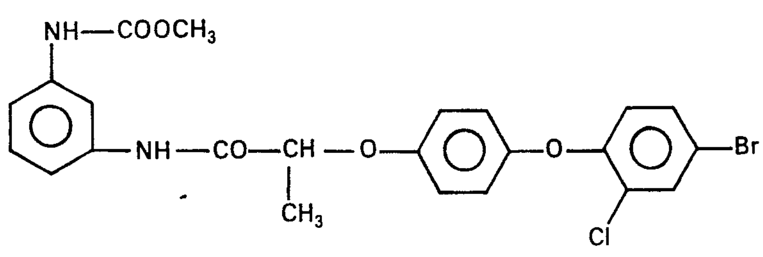

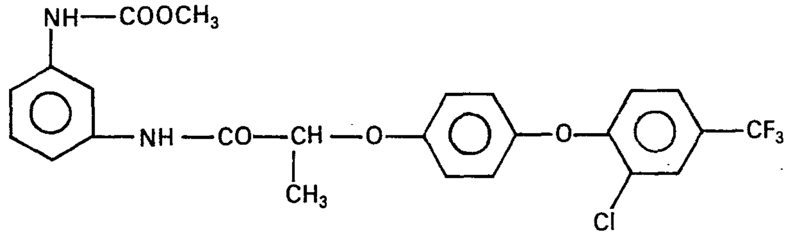

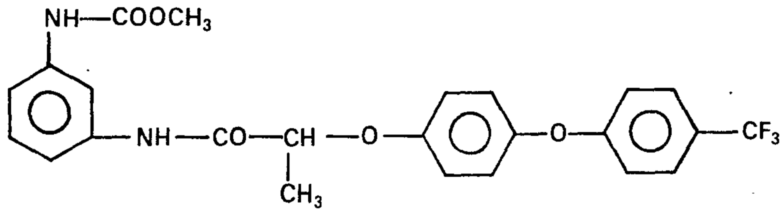

et

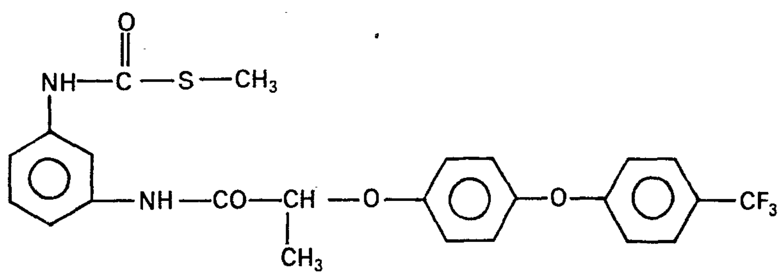

**Revendications pour l'Etat contractant: AT**

1. Herbicide, contenant comme composant un m-anilidurethane de formule générale

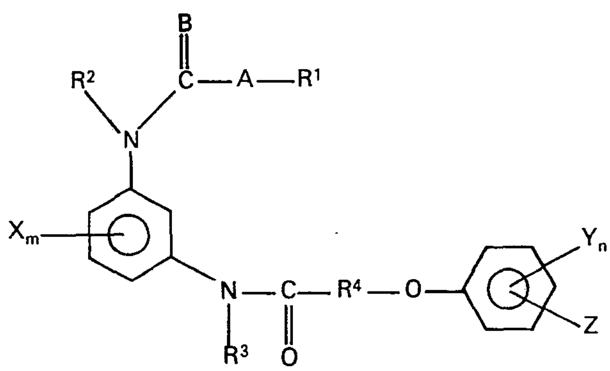

dans laquelle A et B représentent, indépendamment l'un de l'autre, oxygène ou soufre, $R^1$ alkyle, éventuellement substitué par halogène, alcoxy, alcoxycarbonyle ou cyano, ou alcényle, éventuellement substitué par halogène, ou alcinyle, éventuellement substitué par halogène ou alcoxy, ou cycloalkyl, éventuellement substitué par alkyle, ou aryle, $R^2$ et $R^3$, chacun, indépendamment l'un de l'autre, hydrogène, alkyle, alcoxyalkyle, halogénalkyle, $R^4$ un reste alkylène à 1 à 6 atomes de carbone, éventuellement substitué par alkyle, alcoxyalkyle ou halogénalkyle, X hydrogène, alkyle, halogénalkyle, alcoxy, halogène, nitro ou amino, Y hydrogène, alkyle, halogénalkyle, alcoxyalkyle, cycloalkyle, aralkyle, halogène, alcoxy, halogénalcoxy, alkylthio, nitro, aryle, thiocyanato, cyano,

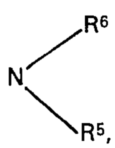 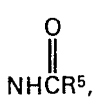 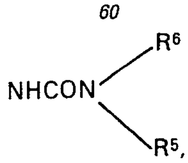 COOR⁵, 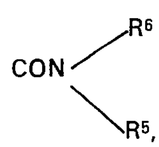

$SO_2R^5$, $OSO_2R^5$, $COR^5$, 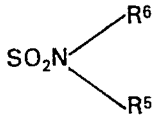

$R^5$ et $R^6$ représentant, indépendamment l'un de l'autre, hydrogène ou ayant la signification donnée pour $R^1$,

Z le reste 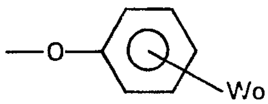 ou 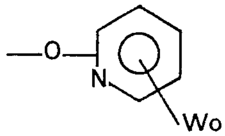

W ayant, indépendamment de Y, la signification donnée pour Y, et m, n et o représentant des nombres de 1 à 4.

2. Herbicide contenant un support solide ou liquide et un m-aniliduréthane de formule générale

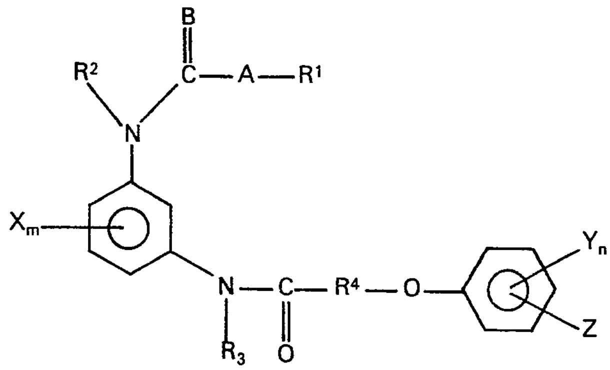

dans laquelle
A et B représentent, indépendamment l'un de l'autre, oxygène ou soufre,
$R^1$ alkyle, éventuellement substitué par halogène, alcoxy, alcoxycarbonyle ou cyano, ou alcényle, éventuellement substitué par halogène, ou alcinyle, éventuellement substitué par halogène ou alcoxy, ou cycloalkyl, éventuellement substitué par alkyle, ou aryle,
$R^5$ et $R^3$, chacun, indépendamment l'un de l'autre, hydrogène, alkyle, alcoxyalkyle, halogénalkyle,
$R^4$ un reste alkylène à 1 à 6 atomes de carbone, éventuellement substitué par alkyle, alcoxyalkyle ou halogénalkyle,
X hydrogène, alkyle, halogénalkyle, alcoxy, halogène, nitro ou amino,
Y hydrogène, alkyle, halogénalkyle, alcoxyalkyle, cycloalkyle, aralkyle, halogène, alcoxy, halogénalcoxy, alkylthio, nitro, aryle, thiocyanato, cyano,

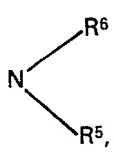 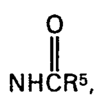 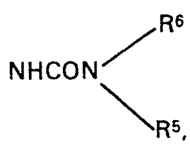 $COOR^5$, 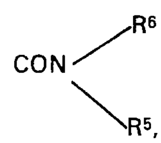

$SO_2R^5$, $OSO_2R^5$, $COR^5$, 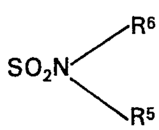

$R^5$ et $R^6$ représentant, indépendamment l'un de l'autre, hydrogène ou ayant la signification donnée pour $R^1$,

Z le reste 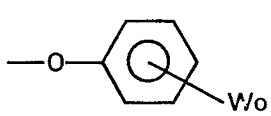 ou 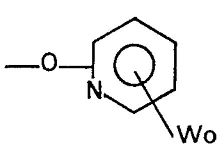

W ayant, indépendamment de Y, la signification donnée pour Y, et m, n et o représentent des nombres de 1 à 4.

3. Procédé de lutte contre la croissance indésirable des plantes, caractérisé par le fait qu'on traite le sol ou les plantes avec un m-anilidurétha-ne de formule générale

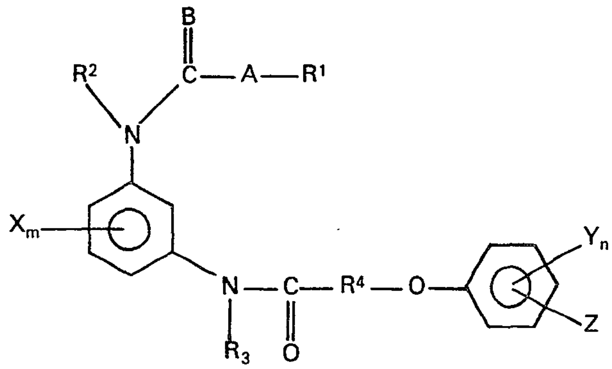

dans laquelle
A et B représentent, indépendamment l'un de l'autre, oxygène ou soufre,
$R^1$ alkyle, éventuellement substitué par halogène, alcoxy, alcoxycarbonyle ou cyano, ou alcényle, éventuellement substitué par halogène, ou alcinyle, éventuellement substitué par halogène ou alcoxy, ou cycloalkyle, éventuellement substitué par alkyle, ou aryle,
$R^2$ et $R^3$, chacun, indépendamment l'un de l'autre, hydrogène, alkyle, alcoxyalkyle, halogénalkyle,
$R^4$ un reste alkylène à 1 à 6 atomes de carbone, éventuellement substitué par alkyle, alcoxyalkyle ou halogénalkyle,
X hydrogène, alkyle, halogénalkyle, alcoxy, halogène, nitro ou amino,
Y hydrogène, alkyle, halogénalkyle, alcoxyalkyle, cycloalkyle, aralkyle, halogène, alcoxy, halogénalcoxy, alkylthio, nitro, aryle, thiocyanato, cyano,

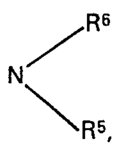 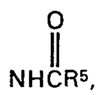 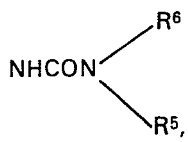 $COOR^5$, 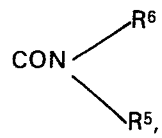

$SO_2R^5$, $OSO_2R^5$, $COR^5$, 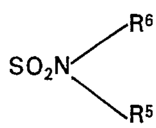

$R^5$ et $R^5$ représentant, indépendamment l'un de l'autre, hydrogène ou ayant la signification donnée pour $R^1$,

Z le reste 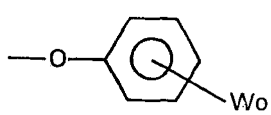 ou 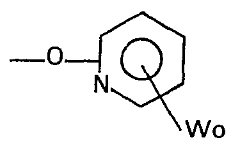

W ayant, indépendamment de Y, la signification donnée pour Y, et m, n et o représentant des nombres de 1 à 4.

4. Herbicide selon la revendication 1 contenant un m-aniliduréthane choisi dans le groupe constitué par les composés de formule

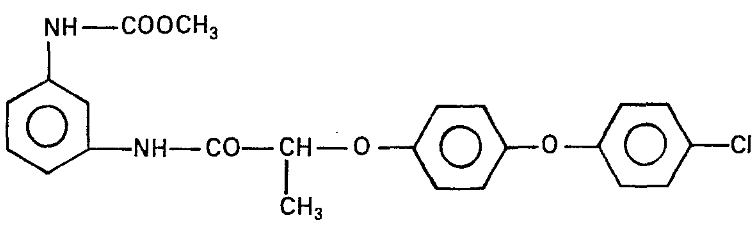

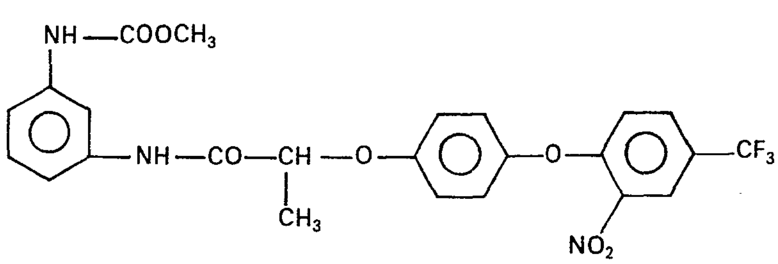

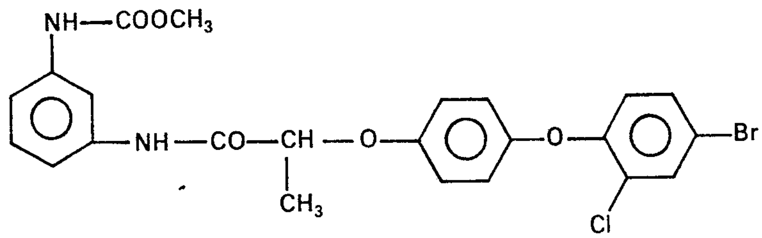

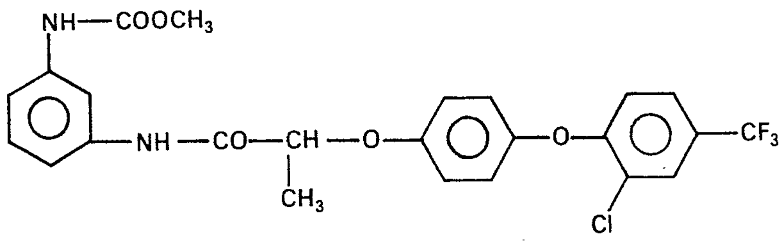

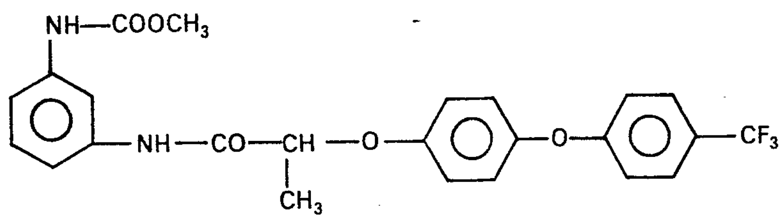

et